(19) Europäisches Patentamt
European Patent Office
Office européen des brevets

(11) **EP 3 617 709 A1**

(12) **EUROPÄISCHE PATENTANMELDUNG**

(43) Veröffentlichungstag:
**04.03.2020   Patentblatt 2020/10**

(51) Int Cl.:
***G01N 33/84*** *(2006.01)*

(21) Anmeldenummer: **18191474.8**

(22) Anmeldetag: **29.08.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Geomar Helmholtz-Zentrum für Ozeanforschung Kiel**
**24148 Kiel (DE)**

(72) Erfinder: **Prof. Dr. Eisenhauer, Anton**
**24226 Heikendorf (DE)**

(74) Vertreter: **RCD Patent**
**Giesen, Schmelcher & Griebel**
**Patentwanwälte PartG mbB**
**Kaiserstraße 100**
**52134 Herzogenrath (DE)**

(54) **DIAGNOSEMETHODE FÜR ERKRANKUNGEN DIE MIT EINER VERMINDERTEN KNOCHENDICHTE EINHERGEHEN**

(57)    Die Erfindung betrifft ein Verfahren zur Diagnose einer Erkrankung, die mit einer verminderten Knochendichte und/oder Calcium Verlust einhergeht, umfassend folgende Schritte:

a) Bestimmen von Isotopenverhältnissen oder Mengenverhältnissen von Erdalkalimetall-Anteilen in einer Probe von Urin oder Blut oder Stuhl,

b) hierdurch Ermitteln mindestens eines Probenwertes, und

c) Vergleich des mindestens einen Probenwertes mit einem Schwellenwert, wobei der Schwellenwert unabhängig gewählt ist von einem oder mehreren durch den individuellen Patienten bedingten Faktoren,

sowie ein Testkit zur Verwendung in der Durchführung des Verfahrens.

EP 3 617 709 A1

**Beschreibung**

Die Erfindung betrifft die Diagnose und/oder dem Ausschluss von Erkrankungen die mit einer verminderten Knochendichte einhergehen, wie beispielsweise Osteoporose, Osteomalazie, Multiples Myelom und/oder Nierenfunktionsstörungen.

Erkrankungen die mit einer verminderten Knochendichte einhergehen, wie beispielsweise Osteoporose, Osteomalazie, Multiples Myelom und/oder Nierenfunktionsstörungen stellen weltweit ein großes Problem dar. Allein die Osteoporose zählt laut WHO zu den 10 häufigsten Erkrankungen.

Die Osteoporose tritt dabei verstärkt ab einem bestimmten Alter und bei Frauen etwa viermal häufiger als bei Männern auf.

In Deutschland ist bei den Hauptdiagnosen von Osteoporose mit pathologischer Fraktur in den Jahren 2010 bis 2016 sogar ein Anstieg von mehr als 20 % zu verzeichnen.

Daher scheint es sehr sinnvoll, insbesondere bei der betroffenen Risikogruppe vermehrt Diagnosen (Reihenuntersuchungen) durchzuführen, um die Gefahr einer sich entwickelnden Osteoporose frühzeitig zu erkennen und entgegenwirkende Maßnahmen zu ergreifen. Auch könnte so die Gefahr einer nicht erkannten Osteoporose und den möglicherweise daraus folgenden gesundheitlichen Schäden (Knochenbrüche, Haltungsschäden) minimiert werden.

Auch angesichts der hohen Anzahl an Erkrankungen wäre hier ein Screeningverfahren, welches im Rahmen der Vorsorgemedizin eingesetzt werden könnte und so sehr frühzeitig nicht Betroffene von den möglicherweise betroffenen Patienten trennen kann, sehr wünschenswert.

Es wäre ebenfalls sehr sinnvoll, therapiebegleitende Diagnosen durchzuführen, um hier eine nicht wie gewünscht verlaufende Therapie gegen Erkrankungen die mit einer verminderten Knochendichte einhergehen, zu erkennen und im Idealfall auch mehr über die Ursachen der Erkrankung zu erfahren und so geeignetere Maßnahmen ergreifen zu können.

Das zurzeit angewendete Standard-Verfahren zur Diagnose und Kontrolle einer Osteoporose und anderer Knochenstoffwechselstörungen mit erhöhtem Risiko eines Knochenbruchs ist das röntgenbasierte DXA oder auch DEXA (Dual-energy X-ray absorptiometry). Nachteilig ist hier die resultierende Strahlungsbelastung für den Patienten und die schlechte Vergleichbarkeit der ermittelten Werte, es können daher im Befund keine absoluten Werte angegeben werden, sondern es wird nur eine Abweichung vom alters- und geschlechtsspezifischen Durchschnitt bestimmt und mit der Standardabweichung verglichen.

Die US 2013/0115650 A1 schlägt vor, das Verhältnis von $^{44}Ca/^{42}Ca$ als Biomarker zur Diagnose von Erkrankungen, die mit einer Veränderung des Mineralstoffhaushaltes des menschlichen Skeletts einhergehen, zu nutzen. Für eine Aussage muss allerdings zunächst eine Basislinie für jedes Individuum bestimmt werden (erste Probe) kurz nach Ansetzen einer Therapie wird eine weitere Probe genommen und vermessen.

Skulan, Joseph; et al; "Natural Calcium Isotopic Composition of Urine as a Marker of Bone Mineral Balance" Clinical Chemistry, 53, 1155-1158 (2007), schlagen vor, über einen längeren Zeitraum zu verschiedenen Zeitpunkten das Calcium-Isotopen-Verhältnis $\delta^{44/40}Ca$ im Urin zu messen, um den Mineralstoffhaushaltes des Skeletts zu überwachen und möglicherweise vorhandenen "Knochenschwund" durch eine längere Bettlägerigkeit zu detektieren.

Die US 2014/0273248 A1 hat die Überprüfung des Mineralstoffhaushaltes des menschlichen Skeletts bei Krebspatienten zum Inhalt. Auch hier muss zunächst eine Basislinie ermittelt werden, anschließend werden nach Beginn einer Therapie, bestehend aus der Gabe von Aromatasehemmern, weitere Proben genommen.

Zum Einsatz in der Vorsorgemedizin, insbesondere auch im Rahmen eines Screeningverfahrens, sind Diagnose-Verfahren, die eine Normierung auf den individuellen Patient durch mehrfache Messung (z.B. durch Aufnehmen einer Basislinie) erfordern, eher ungeeignet.

Es ist daher **Aufgabe** der Erfindung ein Diagnoseverfahren für Erkrankungen die mit einer verminderten Knochendichte einhergehen zur Verfügung zu stellen, bei dem auf eine den Patienten belastende Röntgenstrahlung und eine aufwendige Normierung auf den individuellen Patienten, zum Beispiel mittels einer Basislinie, verzichtet werden kann.

Die Aufgabe der Erfindung wird gelöst durch ein Verfahren zur Diagnose einer Erkrankung, die mit einer verminderten Knochendichte und/oder Calcium Verlust einhergeht, umfassend folgende Schritte:

a) Bestimmen von Isotopenverhältnissen oder Mengenverhältnissen von Erdalkalimetall-Anteilen in einer Probe von Urin oder Blut oder Stuhl,
b) hierdurch Ermitteln mindestens eines Probenwertes, und
c) Vergleich des mindestens einen Probenwertes mit einem Schwellenwert,

wobei der Schwellenwert unabhängig gewählt ist von einem oder mehreren durch den individuellen Patienten bedingten Faktoren.

Die Probenwerte werden dabei in zuvor entnommenen Blut- und/oder Urin- und/oder Stuhlproben bestimmt.

Unter dem Begriff, dass der Schwellenwert unabhängig gewählt ist von einem oder mehreren durch den individuellen Patienten bedingten Faktoren, wird im Sinne der vorliegenden Erfindung insbesondere verstanden, dass weder zuvor eine Patienten-bezogene Basislinie bezüglich der zu ermittelnden Probenwerte bestimmt werden muss, noch weitere Faktoren wie beispielsweise ein vor der eigentlichen Diagnose einzuhaltender Ernährungsplan oder ähnliche Faktoren oder Messungen von vorherigen Werten des einzelnen Patienten durchgeführt werden und auch nicht in das Diagnoseverfahren einbezogen werden. Mit anderen Worten ist der Schwellenwert, je noch Probenart der Urin-, Blut- oder Stuhlprobe, ein fester numerischer Wert.

Vorteilhafterweise kann nunmehr erstmals ein Verfahren zur Diagnose von Erkrankungen, die mit einer verminderten Knochendichte und/oder Calcium Verlust einhergehen, zur Verfügung gestellt werden, das sowohl deutlich in der Handhabung vereinfacht ist, als auch dadurch in der Vorsorge eingesetzt werden kann. Durch den erfindungsgemäßen Wegfall von vorherigen Bestimmungen von Basislinien und anderen Einflussfaktoren wie Ernährungskontrolle und Ernährungsprotokolle, die durch den einzelnen Patienten bisher zu erbringen werden noch bevor die eigentliche Diagnose durchgeführt wurde, ist eine erhebliche Erleichterung in der Durchführung und in der Anwendung des Verfahrens gegeben, die ein weite Verbreitung zulässt und in der Vorsorgediagnose insbesondere für ältere Frauen eingesetzt werden kann, auch ohne Vorverdacht. Eine deutliche Kostenreduktion für das vereinfachte Verfahren ist zudem ein wichtiger Faktor in der weiteren Anwendung und Verbreitung, so dass für eine viel größere Patientengruppe erstmals ein solches "Screening" zur Verfügung steht.

Bei den Probenwerten handelt es sich um ermittelte Isotopenverhältnisse oder Mengenverhältnisse von Erdalkalielementen. Insbesondere handelt es sich bei den Werten um Isotopenverhältnisse des Calciums (Ca) oder um das Mengenverhältnis der Erdalkalielemente von Calcium (Ca) zu Strontium (Sr).

Bevorzugt handelt es sich bei den Probenwerten um massenspektrometrisch ermittelte Isotopenverhältnisse oder Mengenverhältnisse von Erdalkalielementen.

In einer bevorzugten Ausführungsform des Verfahrens werden in Schritt a) die Isotopenverhältnisse des Calciums (Ca) bestimmt.

In einer weiteren bevorzugten Ausführungsform des Verfahrens wird in Schritt a) das Mengenverhältnis der Erdalkalielemente von Calcium (Ca) zu Strontium (Sr) bestimmt.

In einem Aspekt der Erfindung dient in Schritt c) ein vom Individuum unabhängiger fester Schwellenwert, welcher je nach Probenart vorher festgelegt wurde, als Vergleichswert.

In einem Aspekt der Erfindung liegt der Schwellenwert in Schritt c) für das Isotopenverhältnis $^{44}Ca/^{42}Ca$ in einer Blutprobe nach einer $\delta$-Notation bei $\delta^{44/42}Ca_{Blut}$ = -0,85±0,06 ‰.

In einem anderen Aspekt der Erfindung liegt der Schwellenwert in Schritt c) für das Isotopenverhältnis $^{44}Ca/^{42}Ca$ in einer Urinprobe nach einer $\delta$-Notation bei $\delta^{44/42}Ca_{Urin}$ = 0,23±0,06 ‰.

Der Schwellenwert kann dabei entsprechend der Formel:

$$\delta^{m3/m2}Ca = \delta^{m3/m1}Ca \times \left[\frac{ln(m3/m2)}{ln(m3/m1)}\right]$$

mit m3 > m2 > m1, wobei m3, m2, m1 die Massenzahlen repräsentieren, auf andere Isotopenverhältnisse des Calciums übertragen werden.

In einem weiteren Aspekt der Erfindung liegt der Schwellenwert in Schritt c) für das Mengenverhältnis der Erdalkalielemente von Calcium (Ca) zu Strontium (Sr) bei 1772±250 $mol_{ca}/mol_{Sr}$.

In einer bevorzugten Ausführungsform der Erfindung wird in Schritt b) ein Probenwert als $\delta^{44/42}Ca_{Urin}$ und ein weiterer Probenwert als $\delta^{44/42}Ca_{Blut}$ ermittelt und in Schritt c) die Differenz der beiden Probenwerte mit einem Schwellenwert vergleichen, um zusätzlich eine Aussage über die Nierenfunktion zu treffen.

In einer weiteren bevorzugten Ausführungsform der Erfindung werden in Schritt b) ein Probenwert als $\delta^{44/42}Ca_{Stuhl}$ und ein weiterer Probenwert als $\delta^{44/42}Ca_{Blut}$ ermittelt und in Schritt c) die Differenz der beiden Probenwerte mit einem Schwellenwert verglichen, um zusätzlich eine Aussage über die Darmfunktion zu treffen.

In einem Aspekt der Erfindung handelt es sich bei der Erkrankung, die mit einer verminderten Knochendichte und/oder Calcium Verlust einhergeht, um Osteoporose, Osteomalazie, Multiples Myelom und/oder eine Nierenfunktionsstörung.

In einer bevorzugten Ausführungsform des Verfahrens werden die Mengenverhältnisse in Schritt a) als Molverhältnisse der Erdalkalimetall-Anteile in der Probe oder als Massenverhältnisse der Erdalkalimetall-Anteile in der Probe bestimmt.

Eine weitere bevorzugte Ausführungsform des Verfahrens ist dadurch gekennzeichnet, dass wenn in Schritt b) ein Probenwert als $\delta^{44/42}Ca_{Urin}$ bestimmt wird und in Schritt c) der Vergleich mit dem Schwellenwert nach einer $\delta$-

Notation von $\delta^{44/42}Ca_{Urin}$ = 0,23±0.06 ‰ ergibt, dass der Probenwert größer ist, so kann das Vorliegen einer Osteoporose-Erkrankung als unwahrscheinlich ausgeschlossen werden.

Eine weitere bevorzugte Ausführungsform des Verfahrens ist dadurch gekennzeichnet, dass wenn in Schritt b) ein Probenwert als $\delta^{44/42}Ca_{Blut}$ bestimmt wird und in Schritt c) der Vergleich mit dem Schwellenwert nach einer $\delta$-Notation von $\delta^{44/42}Ca_{Blut}$ = -0.85±0.06 ‰ ergibt, dass der Probenwert größer ist, so kann das Vorliegen einer Osteoporose-Erkrankung als unwahrscheinlich ausgeschlossen werden.

Bei einem Aspekt der Erfindung handelt es sich um einen Test bei dem durch Messung des Isotopenverhältnisses von Calciumisotopen bestimmt werden kann, ob bei postmenopausalen Frauen keine Erkrankungen die mit einer verminderten Knochendichte einhergeht, wie z.B. Osteoporose, Osteomalazie und/oder Multiples Myelom vorliegt oder ob eine erhöhte Wahrscheinlichkeit für eine positive Diagnose einer dieser Erkrankungen vorhanden ist.

In einem weiteren Aspekt der Erfindung wird dabei in einer zuvor entnommenen Blutprobe das Isotopenverhältnis $^{44}Ca/^{42}Ca$ bestimmt und nach einer $\delta$-Notation auf einen internationalen Standard normiert.

Die $\delta^{m3/m2}Ca$ Werte lassen sich dabei nach der folgenden Formel auch auf andere Ca-Isotopenverhältnisse (beispielsweise unter Beteiligung von $^{40}Ca$, $^{41}Ca$, $^{46}Ca$, $^{48}Ca$, $^{43}Ca$) übertragen:

$$\delta^{m3/m2}Ca = \delta^{m3/m1}Ca \times \left[\frac{ln(m3/m2)}{ln(m3/m1)}\right]$$

Mit m3 > m2 > m1

Erfindungsgemäß wird der so ermittelte Wert mit einem Schwellenwert (für $\delta^{44/42}Ca_{Blut}$: -0.85±0.06 ‰ verglichen, liegen die Werte oberhalb dieses Schwellenwertes so liegt keine Erkrankungen die mit einer verminderten Knochendichte einhergeht, wie z.B. Osteoporose, Osteomalazie und/oder Multiples Myelom vor, liegen die Werte unterhalb des Schwellenwerts, so besteht ein erhöhtes Risiko einer positiven Diagnose einer Erkrankungen die mit einer verminderten Knochendichte einhergeht.

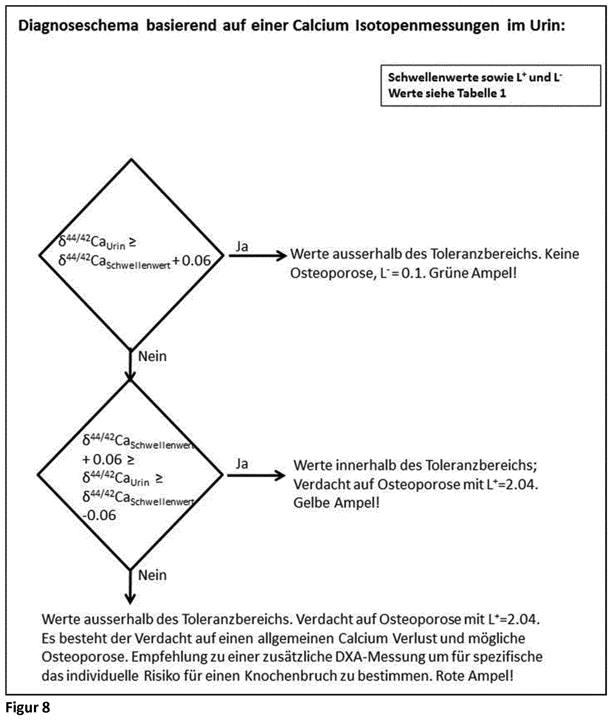

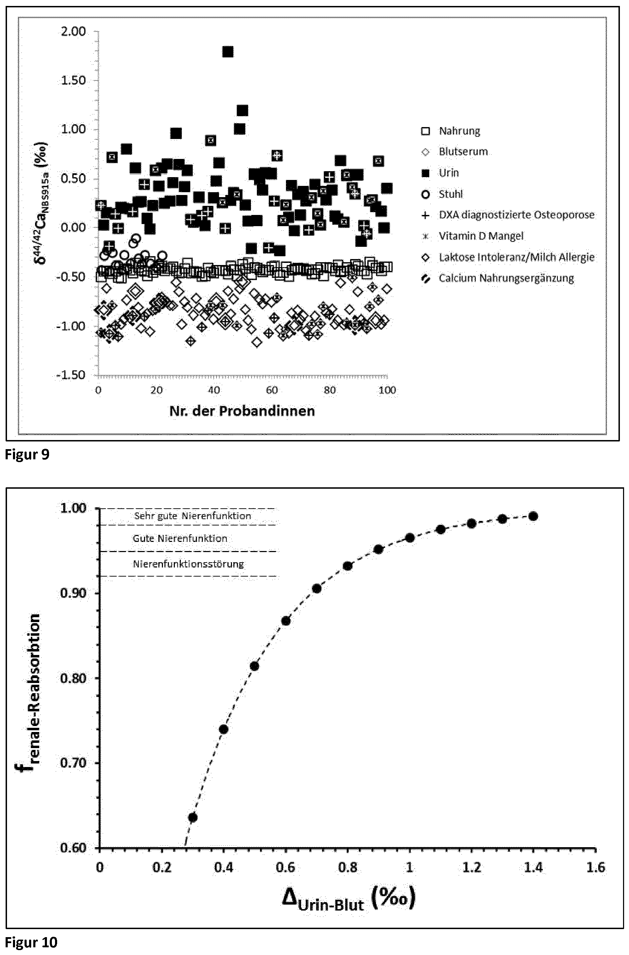

Es ist somit möglich anhand einer einzigen Probe zu einem Diagnose-Resultat zu kommen. Der Schwellenwert von $\delta^{44/42}Ca_{Blut}$ = -0.85±0.06 ‰ wurde anhand einer registrierten (NCT02967978) den Helsinki Kriterien "for good clinical practice" folgenden Studie an der 100 weibliche postmenopausale Probandinnen, die zwischen 50 und 75 Jahren alt waren, ermittelt (Osteogeo Studie). Aufgrund der Variation von Messwerten ist der Wert mit einer statistischen Unsicherheit von ±0.06 ‰ zu versehen, d.h. von -0.79 ‰ bis -0.91 ‰ besteht eine messtechnische bedingte Unsicherheit ob die Patientin unter Knochenschwund leidet oder nicht. Letzteres ist bei der Befundung bzw. Diagnosestellung zu berücksichtigen (siehe Abbildungen 8, 9 und 10).

Der Schwellenwert für $\delta^{44/42}Ca_{Blut}$ lässt sich anhand der folgenden Formel ebenfalls auf andere Ca-Isotopenverhältnisse (beispielsweise unter Beteiligung von $^{40}Ca$, $^{41}Ca$, $^{46}Ca$, $^{48}Ca$, $^{43}Ca$) übertragen:

$$\delta^{m3/m2}Ca = \delta^{m3/m1}Ca \times \left[\frac{ln(m3/m2)}{ln(m3/m1)}\right]$$

Mit m3 > m2 > m1

Das Ergebnis ist sehr aussagekräftig, die Sensitivität des Verfahrens liegt bei 94.4 % der negative Likelihoodfaktor liegt bei 0.1, das bedeutet Frauen deren gemessenen Werte oberhalb des Schwellenwertes liegen, haben relativ zu einer gesunden Person nur eine 0.1-fache Wahrscheinlichkeit einen positiven Osteoporose-Befund zu bekommen.

Es konnte vor der Studie nicht erwartet werden, dass eine Diagnose nur anhand eines Wertes und mit dieser Aussagekraft möglich sein könnte, es musste vielmehr angenommen werden, dass auch der Calcium Isotopen Gehalt der aufgenommenen Nahrung einen Einfluss auf das Ergebnis haben wird. Dieses würde bedeuten, dass eine Normierung auf die einzelne Person durch Messung verschiedener Proben einer Person und/oder gegebenenfalls mehrere Messungen bei Einhalten einer Diät, welche den Calcium Isotopen Gehalt der Nahrung kontrolliert, notwendig wäre.

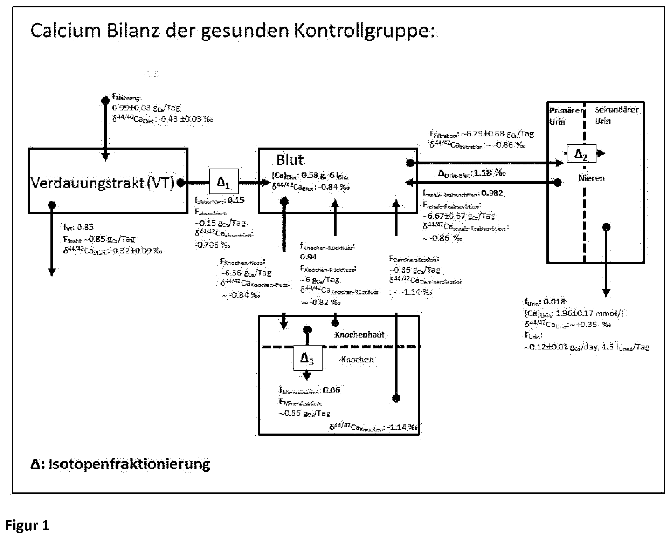

Die Abbildung 1 zeigt die gemessene Calcium Isotopie der Nahrung, des Stuhls, des Blutes und des Urins der 100 postmenopausalen Frauen aus der Osteogeo Studie. Obwohl das Ernährungsverhalten der 100 Frauen individuell sehr stark schwankt ist dessen Calcium Isotopengehalt sehr konstant und kann mit $\delta^{44/42}Ca$: -0.43±0.05 ‰ in engen Grenzen abgeschätzt werden. Daraus ergibt sich auch, dass eine detaillierte Abfrage der zu sich genommenen Nahrung bei einer Diagnose nicht zwingend notwendig ist, da von einem allgemein bekannten Wert ausgegangen werden kann, auch wenn eine besondere Ernährungssituation wie vegetarisch oder vegan vorliegt.

Des Weiteren konnte durch die Osteogeo Studie gezeigt werden, dass der Einfluss des Calciums aus der Nahrung auf die Calcium Isotopenzusammensetzung des Blutes gering ist, da die Absorptionsrate von Calcium aus dem Darm maximal 30 % bis zu ca. 10 % beträgt. Das heißt von dem täglich aufgenommenen Calcium in der Größenordnung von ca. 1 Gramm Calcium pro Tag (gca/Tag) werden nur ca. 0.3 bis 0.15 g absorbiert. Dies entspricht aber nur ca. 2.2 % bis 1.1 % der täglich in die Blutbahn eingetragenen Menge an Calcium (13.18 g Ca/ Tag) aus den Nieren (ca. 6.67 g Ca/ Tag, ca. 50 %) und aus den Knochen (6.36 g Ca/Tag, ca. 48%). Daraus folgt, dass eventuelle isotopische Variationen des Calciums in der Nahrung aufgrund der Massenbilanz gering bis vernachlässigbar sind.

Obwohl die Calcium Isotopie der Nahrung für alle Probandinnen der Studie nahezu konstant war sind die Calcium Isotopenwerte für Blut, Urin und Stuhl aufgrund körpereigener Fraktionierungsprozesse zwischen den verschiedenen Organen unterschiedlich.

Die im Rahmen der Osteogeo Studie ermittelten Calcium-Flüsse zwischen den verschiedenen Organen bei gesunden postmenopausalen Frauen können grafisch folgendermaßen dargestellt werden: siehe Figur 1.

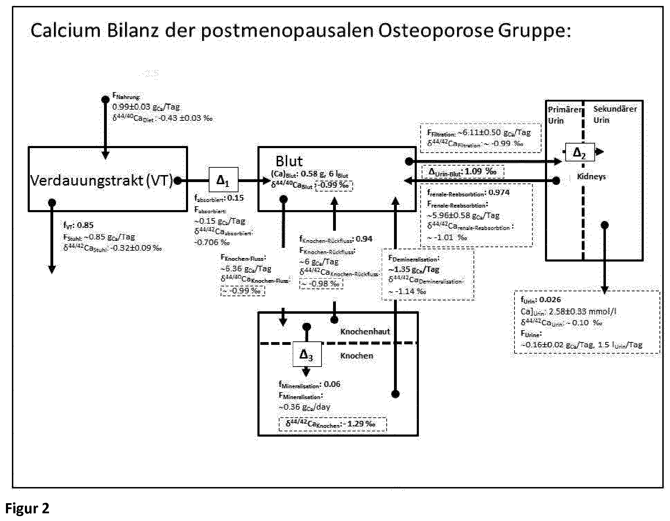

Die folgende Grafik gemäß Figur 2 zeigt die ebenfalls ermittelten Calcium-Flüsse zwischen den Organen bei postmenopausalen Frauen die an Osteoporose leiden. Die Unterschiede in den Calcium Flüssen zwischen den beteiligten Kompartimenten sind mittels einer unterbrochenen Linie markiert.

Die Erfindung hat nun erkannt, dass sich diese tiefgreifenden Erkenntnisse über die Calcium Flüsse zwischen den Organen bei gesunden und an Osteoporose erkrankten Frauen in ein Test-Verfahren umsetzen lässt, bei dem auf das nicht Vorliegen einer Erkrankungen die mit einer verminderten Knochendichte einhergeht, wie z.B. Osteoporose, Osteomalazie und/oder Multiples Myelom (Negativ-Test) und gleichzeitig auf erhöhte Wahrscheinlichkeit eine positive Diagnose der genannten Krankheiten zu erhalten, getestet werden kann. Es ist somit möglich, anhand einer einzigen Blut-Probe zu einem Diagnose-Resultat zu kommen.

Bei einem weiteren Aspekt der Erfindung handelt es sich um einen Test bei dem durch Messung des Isotopenverhältnisses von Calciumisotopen in einer zuvor entnommenen Urinprobe bestimmt werden kann, ob bei postmenopausalen Frauen keine Erkrankungen die mit einer verminderten Knochendichte einhergeht, wie z.B. Osteoporose, Osteomalazie und/oder Multiples Myelom vorliegt oder ob eine erhöhte Wahrscheinlichkeit für eine positive Diagnose einer dieser Erkrankungen vorhanden ist.

Es wird dabei in einer zuvor entnommenen Urinprobe das Isotopenverhältnis $^{44}Ca/^{42}Ca$ bestimmt und nach einer δ-Notation auf einen internationalen Standard normiert.

Erfindungsgemäß wird der so ermittelte Wert mit einem Schwellenwert (für $\delta^{44/42}Ca_{Urin}$ 0.23±0.06 ‰ verglichen, liegen die Werte oberhalb dieses Schwellenwertes so liegt keine Osteoporose vor, liegen die Werte unterhalb des Schwellenwerts, so besteht ein erhöhtes Risiko einer positiven Osteoporose-Diagnose.

Es ist somit möglich anhand einer einzigen Probe zu einem Diagnose-Resultat zu kommen. Der Schwellenwert von $\delta^{44/42}Ca_{Urin}$ 0.23±0.06 ‰ wurde anhand einer Studie an der 100 weibliche Probandinnen, die zwischen 50 und 75 Jahren alt waren, ermittelt.

Der Schwellenwert für $\delta^{44/42}Ca_{Urin}$ lässt sich anhand der folgenden Formel auch auf andere Ca-Isotopenverhältnisse (beispielsweise unter Beteiligung von $^{40}Ca$, $^{41}Ca$, $^{46}Ca$, $^{48}Ca$, $^{43}Ca$) übertragen:

$$\delta^{m3/m2}Ca = \delta^{m3/m1}Ca \times \left[\frac{ln(m3/m2)}{ln(m3/m1)}\right]$$

Mit m3 > m2 > m1

Die Sensitivität des Verfahrens liegt bei 72.2 % der negative Likelihoodfaktor liegt bei 0.41, das bedeutet Frauen deren gemessene Werte oberhalb des Schwellenwertes liegen, haben nur eine 0.41-fache Wahrscheinlichkeit relativ zu einer gesunden Person einen positiven Osteoporose-Befund zu bekommen.

Es konnte vor der Studie nicht erwartet werden, dass eine Diagnose nur anhand eines Wertes möglich sein könnte, es musste vielmehr angenommen werden, dass auch der Calcium Isotopen Gehalt der aufgenommenen Nahrung einen Einfluss auf das Ergebnis haben wird. Dieses würde bedeuten, dass eine Normierung auf die einzelne Person durch Messung verschiedener Proben einer Person und/oder gegebenenfalls mehrere Messungen bei Einhalten einer Diät, welche den Calcium Isotopen Gehalt der Nahrung kontrolliert, notwendig wäre.

Die Erfindung hat nun erkannt, dass sich diese tiefgreifenden Erkenntnisse über die Calcium Flüsse zwischen den Organen bei gesunden und an Osteoporose erkrankten Frauen in ein Test-Verfahren umsetzen lässt, bei dem auf das nicht Vorliegen einer Erkrankungen die mit einer verminderten Knochendichte einhergeht, wie z.B. Osteoporose, Osteomalazie und/oder Multiples Myelom (Negativ-Test) und gleichzeitig auf erhöhte Wahrscheinlichkeit eine positive Diagnose der genannten Krankheiten zu erhalten, getestet werden kann. Es ist somit möglich, anhand einer einzigen Urin-Probe zu einem Diagnose-Resultat zu kommen.

Die Möglichkeit der Diagnose anhand einer einzigen Probe eines Individuums stellt ein sehr überraschendes Ergebnis dar. Die Existent des Schwellenwertes von $\delta^{44/42}Ca_{Urin}$ 0.23±0.06 ‰ und $\delta^{44/42}Ca_{Blut}$ -0.85±0.06 ‰ konnte nicht erwartet werden.

Bei einem weiteren Aspekt der Erfindung handelt es sich um einen Test bei dem durch Messung des Verhältnis der Erdalkalielemente Calcium (Ca) zu Strontium (Sr) im Urin $(Ca/Sr)_{Urin}$ die Fähigkeit zur Resorption von Ca und damit die Wahrscheinlich des Vorliegens einer Erkrankungen die mit einer verminderten Knochendichte einhergeht, wie z.B. Osteoporose, Osteomalazie und/oder Multiples Myelom, charakterisiert wird. Das Ergebnis ist insbesondere vor dem Hintergrund überraschend, da das Verhältnis von Ca und Magnesium (Mg), ebenfalls ein Erdalkalielement, eben diese Korrelation nicht aufweist.

Aus der ROC-Analyse (Receiver-Operating-Characteristics) konnte ein statistisch signifikanter Zusammenhang der $(Ca/Sr)_{Urin}$-Werte mit den DXA-Werten ermittelt werden. Wobei die DXA-Methode, die zurzeit gültige Standardmethode zu Ermittlung der Knochendichte und der Diagnose von Osteoporose ist.

Es konnte ein $(Ca/Sr)_{Urin}$-Schwellenwert ermittelt werden, der es erlaubt an Osteoporose leidende Patientinnen von gesunden Patientinnen zu unterscheiden. Als Schwellenwert ergab sich $1772 \pm 250$ $mol_{Ca}/mol_{Sr}$ welcher erkrankte von gesunden Patientinnen trennt.

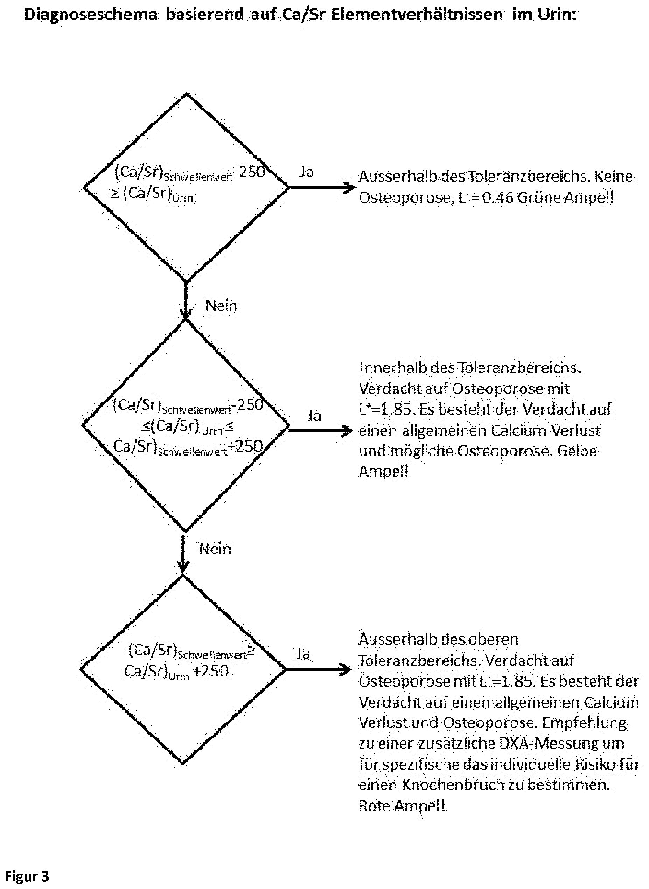

Der Diagnosepfad lässt sich grafisch folgendermaßen gemäß Figur 3 darstellen.

In einem weiteren Aspekt der Erfindung handelt es sich um Verfahren zur Bestimmung der Nierenfunktionalität anhand des Vergleichs der Ca Isotopendifferenz in einer zuvor entnommenen Urin- und Blutprobe.

Es zeigte sich, dass die Ca Isotopendifferenz $\Delta_{Urin-Blut} = \delta^{44/42}Ca_{Urin} - \delta^{44/42}Ca_{Blut}$ die Recycling-Effizienz der Niere für Spurenelemente wiederspiegelt und dass dieser Zusammenhang nicht-linear aber soweit kalkulierbar ist, dass aus den Werten die recycling Rate $f_{renale-Reabsorbtion}$ der Niere berechnet werden kann. (siehe Figuren).

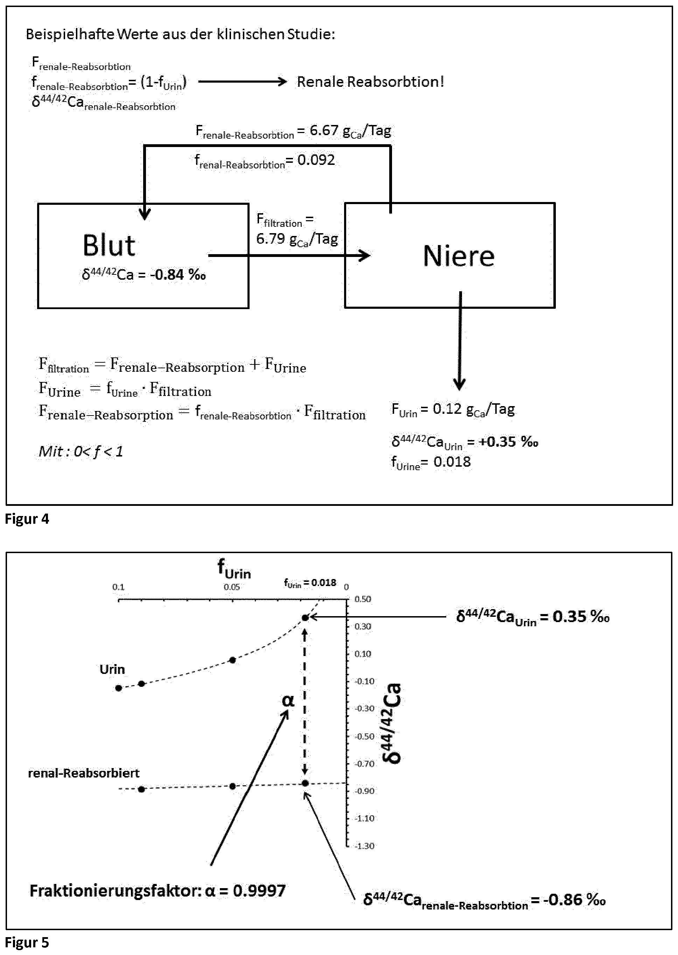

Die folgende Grafik zeigt gemäß Figur 4 die Calcium-Flüsse und Isotopenfraktionierung zwischen dem Blutsystem und den Nieren. Mit Hilfe der Calcium Isotopenverhältnisse gemessen in Blut und Urin kann die Rate der renalen Reabsorbtion des Calciums in der Niere berechnet werden. Letzteres ist ein Maß für die Funktionalität der Nieren.

Die Funktionalität der Nieren kann dann anhand der berechneten Recycling Rate erfindungsgemäß entsprechend Tabelle 1 klassifiziert werden:

Tabelle 1: Klassifikation (Schwellenwerte) der Nierenfunktion mittels der renalen Reabsorbtion ($f_{renale-Reabsorbtion}$)

| | |
|---|---|
| $1.00 > f_{renale-Reabsorbtion} = \geq 0.98$ | → sehr gute Nierenfunktion |
| $0.98 > f_{renale-Reabsorbtion} = \geq 0.95$ | → gute Nierenfunktion |
| $0.95 > f_{renale-Reabsorbtion} = \geq 0.92$ | → Nierenfunktionsstörung |
| $0.92 > f_{renale-Reabsorbtion}$ | → zunehmende Nierenfunktionsstörung |

Die Kalibration der Werte erfolgte dabei durch Bestimmung der eGFR (estimated Glomual Filtration Rate), welche an 12 Patientinnen mit unterschiedlicher Nierenfunktionalität durchgeführt wurde. Zur glomeruläre Filtrationsrate (GFR) ist zu sagen, dass es sich hier um die zur Zeit etablierte Methode zur Einschätzung der Nierenfunktion handelt.

Die GFR wird entweder relativ aufwendig aus 24-h-Sammelurin als Kreatininclearance bestimmt oder mittels verschiedenster Näherungsformeln eGFR aus der Plasmakreatininkonzentration unter Berücksichtigung der Hautfarbe, des Alters, des Geschlechts und noch anderer Größen berechnet.

Am gebäuchlisten ist die sogenannte MDMR Formel [Saulo Klahr, Andrew S. Levey, Gerald J. Beck, Arlene W. Caggiula, Lawrence Hunsicker, John W. Kusek, Gary Striker, The Modification of Diet in Renal Disease Study Group: The Effects of Dietary Protein Restriction and Blood-Pressure Control on the Progression of Chronic Renal Disease. In: N Engl J Med. 330, Nr. 13, 31. März 1994, S. 877-884. doi:10.1056/NEJM199403313301301].

Die Näherungsformeln sind validiert für ambulante, chronisch nierenkranke Patienten mit moderater bis schwerer Nierenfunktionseinschränkung (Stadium 3 und 4). Für leichte Nierenfunktionsstörungen bzw. auch keine Nierenfunktionsstörung sind die Formeln in der Regel nicht anwendbar.

Die Formeln sind ebenfalls nicht geeignet zur Bestimmung der glomerulären Filtrationsrate bei Personen mit:

- normaler Nierenfunktion
- leichter Nierenfunktionseinschränkung.
- Insbesondere die MDRD-Formel unterschätzt bei Menschen mit einer glomerulären Filtrationsrate über 60 ml/min diese um ca. 10 ml/min [Stevens, Lesley A. et al.: Evaluation of the Modification of Diet in Renal Disease Study Equation in a Large Diverse Population. In: J Am Soc Nephrol. Nr. 18, 2007, S. 2749-2757 (asnjournals.org)]
- akuter Nierenfunktionsverschlechterung,
- schwerem Übergewicht
- stark verminderter Muskelmasse (Amputation von Gliedmaßen, Unterernährung)
- mit besonders hoher (Nahrungsergänzungen bei Bodybuildern)
- niedriger Kreatin-Zufuhr mit der Nahrung (Vegetarier).
- zur Überwachung der Nierenfunktion im besonders wichtigen Frühstadium der diabetischen Nephropathie [Nephrology beyond JASN. Eberhard Ritz Feature Editor: Estimated GFR: Are There Limits to Its Utility? J Am Soc Nephrol, 2006, 17, S. 2077-2085]

Erfindungsgemäß wird die Ca Isotopendifferenz $\Delta_{Urin\text{-}Blut} = \delta^{44/42}Ca_{Urin} - \delta^{44/42}Ca_{Blut}$ zugrunde gelegt, um die Recycling-Effizienz der Niere zu klassifizieren. Aus den Werten wird die recycling Rate $f_{renale\text{-}Reabsorbtion}$ der Niere berechnet. Anschließend erfolgt die Klassifizierung anhand der Schwellenwerte aus Tabelle 1.

Die $\delta$ - Notation $\delta^{44/42}Ca$ lässt sich dabei anhand der folgenden Formel auch auf andere Ca-Isotopenverhältnisse (beispielsweise unter Beteiligung von $^{40}Ca$, $^{41}Ca$, $^{46}Ca$, $^{48}Ca$, $^{43}Ca$) übertragen:

$$\delta^{m3/m2}Ca = \delta^{m3/m1}Ca \times \left[\frac{ln(m3/m2)}{ln(m3/m1)}\right]$$

Mit m3 > m2 > m1

Die zur Berechnung relevanten Größen $\delta^{44/42}Ca_{Blut}$, $\delta^{44/42}Cau_{Urin}$ und $F_{Urin}$ sind durch Messung bekannt. Wobei $F_{Urin}$ der tägliche Verlust von Calcium über den Urin angibt.

Die mittels unterbrochener Linien markierten Ca Isotopien und Flüsse können durch die Raleigh Gleichungen und die Isotopenbilanz errechnet werden. Dabei wird folgendermaßen vorgegangen:

Die Definition $\delta$-Notation ist:

$$(01) \qquad \delta^{44/42}Ca_{Blut}\,(‰) = \left[\frac{\left(\frac{^{44}Ca}{^{42}Ca}\right)_{Blut}}{\left(\frac{^{44}Ca}{^{42}Ca}\right)_{Standard}} - 1\right]$$

und für Urin

$$(02) \qquad \delta^{44/42}Ca_{Urin}\,(‰) = \left[\frac{\left(\frac{^{44}Ca}{^{42}Ca}\right)_{Urin}}{\left(\frac{^{44}Ca}{^{42}Ca}\right)_{Standard}} - 1\right]$$

Zur Vereinfachung wir folgendes gesetzt:

$$R_{Blut} = \left(\frac{^{44}Ca}{^{42}Ca}\right)_{Blut} \text{ und } R_{Urin} = \left(\frac{^{44}Ca}{^{42}Ca}\right)_{Urin}$$

Das Verhältnis von $R_{Urin}/R_{Blut}$ ergibt sich dann aus der Gleichung (03):

$$(03) \qquad \frac{R_{Urin}}{R_{Blut}} = \frac{\left(\frac{^{44}Ca}{^{42}Ca}\right)_{Urin}}{\left(\frac{^{44}Ca}{^{42}Ca}\right)_{Blut}} = \frac{\delta^{44/42}Ca_{Urin} - 1}{\delta^{44/42}Ca_{Blut} - 1}$$

Die Gleichung (03) hat auch den Vorteil, dass man das Verhältnis von $R_{Urin}/R_{Blut}$ berechnen kann ohne den absoluten ($^{44}Ca/^{42}Ca$) Standard-Wert zu kennen.

Zur Beschreibung der Sequestrierung eines Ca Flusses und der damit einhergehenden Ca Isotopenfraktionierung wird nun die Formel der Rayleigh Destillation angewendet.

In die Formeln (04) bis (06) der die Destillation beschreibenden Formeln gehen folgende bekannten bekannte (gemessene) Größen ein:

a) das Verhältnis der gemessenen Ca Isotopenwerte des Urin und des Blutes

$$R_{Urin}/R_{Blut} = (\delta^{44/42}Ca_{Urin}-1)/(\delta^{44/42}Ca_{Blut}-1)$$

und folgende unbekannte Größen:

i. Der Verteilungsquotient der die Aufteilung des Ca zwischen Blut und Urin beschreibt ($0 \leq f_{Urin} \leq 1$). Der Ausdruck $f_{Urin}$ beschreibt dabei den relativen Anteil des mit dem Urin ausgeschiedenen Calciums und $f_{renale\text{-}Reabsorbtion}$ = ($1-f_{Urin}$) den Anteil des Calciums welcher in den Blutkreis reabsorbiert wird.

ii. der Fraktionierungsfaktor "$\alpha$" zwischen Blut und Urin

iii. sowie der Ca Isotopenwert $\delta^{44/42}Ca_{renale\text{-}Reabsorbtion}$ des in den Blutkreislauf zurückgeführten Calcium

Zur Anwendung kommen die Gleichungen (04) bis (06).

$$(04) \quad \frac{R_{Urin}}{R_{Blut}} = f_{Urin}^{(\alpha-1)}$$

$$(05) \quad \frac{R_{renale-Reabsorbtion}}{R_{Blut}} = \frac{\delta^{44/42}Ca_{renale-Reabsorbtion}-1}{\delta^{44/42}Ca_{Blut}-1} = \frac{\left(1-f_{Urin}^{\alpha}\right)}{1-f}$$

$$(06) \quad \delta^{44/42}Ca_{Blut} = f_{Urin} \cdot \delta^{44/42}Ca_{Urin} + (1-f_{Urin}) \cdot \delta^{44/42}Ca_{renale-Reabsorbtion}$$

Zur weiteren Vereinfachung und Handhabung der Gleichungen (04) bis (06) kann die sogenannte in der Mathematik gebräuchliche "kleine Näherung" verwenden. Der Vorteil dieser Vorgehensweise ist, dass die gemessenen isotopische Differenz zwischen Blut und Urin ($\Delta_{Urin\text{-}Blut} = \delta^{44/42}Ca_{Urin} - \delta^{44/42}Ca_{Blut}$) eingesetzt werden kann. Weiterhin kann dann eine direkte Beziehung zwischen der isotopischen Urin-Blut Differenz und der Filtrationseffizienz "f" hergestellt werden:

Es gilt ("kleine Näherung"):

$$\ln\left(\frac{R_{Urin}}{R_{Blut}}\right) \cong \frac{\delta^{44/42}Ca_{Urin} - \delta^{44/42}Ca_{Blut}}{1000} \cong \Delta_{Urin-Blut}$$

Die kleine Näherung für ln (x) = x gilt wenn x<<1 ist. In der Isotopenchemie wird dieses für alle Bereiche von ca. -30 bis +40 ‰ eingesetzt. Es gilt dann 1000 ln (x) = $d^1l_a$ - $d^1l_b$ ($d^1l_a$, $d^1l_b$ Isotopenverhältnisse). Es muss mit 1000 multipliziert werden, da die $d^1l_a$ in Promill (‰) angegeben sind.

Daraus folgt:

$$\frac{R_{Urin}}{R_{Blut}} \cong e^{\frac{\Delta_{Urin-Blut}}{1000}};$$

Einsetzen in Gleichung (04) ergibt:

$$(07) \quad \frac{R_{Urin}}{R_{Blut}} = e^{\frac{\Delta_{Urin-Blut}}{1000}} = f_{Urin}^{\alpha-1};$$

In gleicher Weise wird für die Gleichung (05) vorgegangen, so dass dann gilt:

$$(08) \quad \frac{R_{renale-Reabsorbtion}}{R_{Blut}} = e^{\frac{\Delta_{(renale-Reabsorbtion)-Blut}}{1000}} = \frac{\left(1-f_{Urin}^{\alpha}\right)}{1-f}$$

Das zu lösende Gleichungssystem ist folgendes:

$$(09) \quad e^{\frac{\Delta_{Urin-Blut}}{1000}} = f_{Urin}^{(\alpha-1)}$$

$$(10) \quad \frac{\delta^{44/42}Ca_{renale-Reabsorbtion}-1}{\delta^{44/42}Ca_{Blut}-1} = \frac{(1-f_{Urin}^{\alpha})}{1-f}$$

$$(11) \quad \delta^{44/42}Ca_{Blut} = f_{Urin} \cdot \delta^{44/42}Ca_{Urin} + (1-f_{Urin}) \cdot \delta^{44/42}Ca_{renale-Reabsorbtion}$$

Das umgeformte nicht-linearen Gleichungssystem (09) bis (11) ist zu dem in (04) bis (06) identisch, zeigt aber analytisch insbesondere mit Gleichung (09), dass es einen funktionalen nicht-linearen Zusammenhang zwischen der Ca Isotopendifferenz zwischen Blut und Urin und der Recyclingrate "f" der Nieren gibt. Dieses Gleichungssystem hat drei Unbekannte (f, $\alpha$ und $\delta^{44/42}Ca_{renale-Reabsorbtion}$) und kann daher mit den vorhandenen drei Gleichungen gelöst werden. Es handelt es sich aber um ein nicht-lineares System, so dass hier die Regeln der linearen Algebra nicht gelten, sondern iterative Optimierungen für die Suche nach einer simultanen Lösung herangezogen werden müssen.

Die Funktionswerte der Gleichung (04) und (05) oder gleichwertig (09) und (10) lassen sich grafisch darstellen. (Rayleigh Gleichungen, siehe Figur 5)

Die obere Kurve reflektiert die Ca Isotopenzusammensetzung des Urins ($\delta^{44/42}Ca_{Urin}$) als Funktion des im Urin verbleibenden Calciums. Die untere Kurve reflektiert die Ca Isotopie des renal-reabsorbierten ($\delta^{44/42}Ca_{renale-Reabsorbtion}$) und in den Blutkreislauf reabsorbierten Calciums. Der Fraktionierungsfaktor "$\alpha$" ist für die isotopische Differenz und deren quantitativer Größe verantwortlich.

In der Abbildung 2 ist der Zusammenhang zwischen der isotopischen Unterschied zwischen Blut und Urin ($\Delta^{44/42}Ca_{Blut-Urin}$) sowie der Rate der renalen Reabsorbtion ($f_{renale-Reabsorbtion}$) grafisch dargestellt. Zusätzlich sind die Bereiche der Nierenfunktionalität (siehe weiter oben) mit eingezeichnet.

In einem weiteren Aspekt der Erfindung handelt es sich um ein Verfahren zur Bestimmung der individuellen Calcium (Ca) Aufnahme aus dem Darm in das Blut über die Differenz der Ca-Isotopenverhältnisse $\delta^{44/42}Ca$ (Delta-Notation). Bei festgestellter Osteoporose können so insbesondere in Zusammenhang mit den Erkenntnissen aus der Funktionalität der Nieren über die Recycling-Effizienz der Niere $\Delta_{Urin-Blut} = \delta^{44/42}Ca_{Urin} - \delta^{44/42}Ca_{Blut}$, dem behandelnden Arzt wichtige Hinweise gegeben werden, durch welche Probleme die Osteoporose verursacht sein könnte und welche Therapien ratsam sind.

Die Bestimmung erfolgt dabei anhand der Differenz der Calcium-Isotopenverhältnisse im Blut, Stuhl und in der Nahrung. Wobei hier insgesamt mehrere über einen Zeitraum von einigen Tagen entnommenen Proben verwendet werden.

Anhand der in der Osteogeo-Studie erworbenen Erkenntnisse soll die Berechnung der Absorbtionsrate über die Darm-Blutschranke erläutert werden.

Der im Rahmen der Studie gemessene $\delta^{44/42}Ca_{Nahrung}$-Wert beträgt $-0.43\pm0.01$ ‰.

Der gemessene Wert $\delta^{44/42}Ca_{Stuhl}$ des Stuhles beträgt $-0.32\pm0.09$ ‰.

Eine unbekannte relative Calcium Menge ($f_{Blut-Darm}$) wird vom Blut absorbiert ($f_{Blut-Darm}$: $0\leq f_{Blut-Darm} \leq 1$, wobei $f_{Blut-Darm}$ als Absorbtionsrate bezeichnet wird), und eine komplementäre Ca Menge ($1-f_{Blut-Darm}$) wird mit dem Stuhl ausgeschieden. Beim Übergang des Ca aus dem Darm in das Blut kommt es zu einer Isotopenfraktionierung unbekannter Größe (a), so dass der Ca Isotopenwert des absorbierten Ca ($\delta^{44/42}Ca_{absorbiert}$ ebenfalls unbekannt ist. Die drei unbekannten Größen $f_{Blut-Darm}$, $\alpha$, und $\delta^{44/42}Ca_{absorbiert}$ lassen sich mittels dreier nichtlinearer Gleichungen errechnen:

Aus den drei nicht-linearen Gleichungen und den zwei gemessenen bekannten Isotopenwerten ($\delta^{44/42}Ca_{Nahrung}$ und $\delta^{44/42}Ca_{Stuhl}$) können die drei unbekannten Größen $f_{Blut-Darm}$, $\alpha$, und $\delta^{44/42}Ca_{absorbiert}$ berechnet werden:

$$(01) \quad \frac{R_{Stuhl}}{R_{Nahrung}} = f_{Blut-Darm}^{(\alpha-1)}$$

$$(02) \quad \frac{R_{absorbiert}}{R_{Nahrung}} = \frac{(1-f_{Blut-Darm}^{\alpha})}{1-f_{Blut-Darm}}$$

$$(03) \quad \delta^{44/42}Ca_{Nahrung} = f_{Blut-Darm} \cdot \delta^{44/42}Ca_{Stuhl} + (1-f_{Blut-Darm}) \cdot \delta^{44/42}Ca_{absorbiert}$$

"R" ist das gemessene $^{44}Ca/^{42}Ca$ Verhältnis, welches aus der $\delta$-Notation zurück gerechnet werden kann:
Es gilt:

$$\frac{\delta^{44/42}Ca_{Stuhl}}{\delta^{44/42}Ca_{Nahrung}} = \frac{R_{Stuhl}-1}{R_{Nahrung}-1} \text{ und } \frac{\delta^{44/42}Ca_{absorbiert}}{\delta^{44/42}Ca_{Nahrung}} = \frac{R_{Stuhl}-1}{R_{Nahrung}-1}$$

Es handelt es sich aber um ein nicht-lineares System, so dass hier die Regeln der linearen Algebra nicht gelten, sondern iterative Optimierungen für die Suche nach einer simultanen Lösung herangezogen werden müssen. Die durchschnittliche Absorbtionsrate $f_{Blut-Darm}$ liegt, nach Angaben aus der medizinischen Fachliteratur [Heaney RP. The Calcium Economy. p145-162, In: Weaver CM, Heaney RP, editors. Calcium in Human Health. Totowa, New Jersey: Human Press; 2006] bei $f_{Blut-Darm}$ = 0.3 also 30%. In der genannten Literaturstelle wird gezeigt, dass eine Testperson, die 20mmol/d (0.8 gca/Tag) mit der Nahrung und 3.4 mmol/d (0.136 gca/Tag) von den Ca-haltigen Verdauungssäften aufnimmt, 16.6 mmol/d (0.66 gca/Tag) ausscheidet. Berücksichtigt man nur die Nahrung, so ist die Aufnahme 17 %, berücksichtigt man Nahrung und die Ca-haltigen Verdauungssäfte, dann ist die Aufnahme ca. 30 %.

Ein Wert von $f_{Blut-Darm}$ = 0.3 also 30 % kann -bei Berücksichtigung der Ca-haltigen Verdauungssäfte- somit als der Sollwert bei einer durchschnittlichen Ca Aufnahme von ca. 1g Calcium täglich angesehen werden.

Die $\delta$ - Notation $\delta^{44/42}Ca$ lässt sich dabei wie auch in den vorangegangenen Erfindungen anhand der folgenden Formel auch auf andere Ca-Isotopenverhältnisse (beispielsweise unter Beteiligung von $^{40}Ca$, $^{41}Ca$, $^{46}Ca$, $^{48}Ca$, $^{43}Ca$) übertragen:

$$\delta^{m3/m2}Ca = \delta^{m3/m1}Ca \times \left[\frac{ln(m3/m2)}{ln(m3/m1)}\right]$$

Mit m3 > m2 > m1

Im Ergebnis erhält der Arzt so die Erkenntnis, ob die vorliegende Osteoporose durch eine renale Osteopathie (Nierenfunktionsstörung) oder durch eine verminderte Calcium (Ca) Aufnahme aus dem Darm, ausgelöst wurde und somit eine sekundäre Osteoporose vorliegt.

**Material und Methoden:**

Die Allgemeinheit der Lehre nicht einschränkend wird im Folgenden das exemplarische Vorgehen erläutert.

**Probenentnahme für Blut und Urin:**

Dem Patienten wird durch den Arzt eine Blutprobe mit einer normalen für Blutuntersuchungen benötigten Blutmenge (ca. 8 ml) entnommen. Die Blutprobe wird eine halbe Stunde stehen gelassen danach zentrifugiert. Das so entstandene Blutserum wird vom Blutkuchen getrennt. Für die weiteren chemischen Aufbereitungen wird ausschließlich Blutserum verwendet.

Für die weitere chemische Aufbereitung wird eine Blutmenge entnommen die einer absoluten Menge von 50 $\mu$g Calcium entspricht.

Die Urinprobe, ca. 10 ml, kann die Patientin mittels eines dafür vorgesehenen Behältnisses selbst entnehmen. Für die weitere chemische Aufbereitung wird eine Urinmenge entnommen die, wie beim Blut, einer absoluten Menge von 50 $\mu$g Calcium entspricht.

**Extraktion der Erdalkalielemente aus den Proben:**

Einem chemischen Verfahren wird das Calcium aus dem Blut und dem Urin extrahiert bis eine Lösung mit einer Konzentration von ca. 5 ppm zur massenspektrometrischen Messung zur Verfügung steht.

Die Aufbereitung des Blutes zur chemischen Extraktion des Calciums kann nach dem folgenden Verfahren erfolgen:

**Chemische Probenaufbereitung zur Calcium Isotopenbestimmung an Blut und Urin**

**Tag 1**

- Die für den Mikrowellenaufschluss (MW) vorgeschriebenen Gefäße werden mit $HNO_3$ und $H_2O_2$ befüllt.
- Die vorbereiteten Proben und Standards werden zu dem jeweiligen Gefäß pipettiert.
- Gefäße werden verschlossen, das Mikrowellengerät wird gestartet und für 1.5 Stunden in die MW gestellt.
- Die aufgeschlossenen Proben werden aus der MW geholt. Im Abzug werden die Lösungen nacheinander in Becher umgefüllt und auf die Heizplatte zum Trocknen (über Nacht) gestellt.

**Tag 2**

- Die aufgeschlossenen und über Nacht getrockneten Proben werden mit 1ml $HNO_3$ +0.5ml $H_2O_2$ aufgenommen und wieder verschlossen für 3 Stunden gekocht.
- Becher öffnen und Lösung eintrocknen.
- Getrocknete Proben werden in 1ml 2M $HNO_3$ aufgenommen.

**Tag 3**

• Die Konzentration der Erdalkalielemente Strontium (Sr), Calcium (Ca und Magnesium) wird an der Q-ICP-MS mittels Standardverfahren gemessen.

**Tag 4**

- Um für die Säulenchromatographie immer die gleiche absoluten Ca Mengen (50 $\mu g_{Ca}$) zu haben wird die Menge an Säure die für die automatisierte Messung an einer ESI PrepFast benötigt berechnet und die Proben entsprechend verdünnt.
- Die Proben werden aus den Bechern in die PrepFAST® Röhrchen (Fa. Elemental Scientific) überführt.
- Die Abtrennung der Erdalkalielemente erfolgt automatisch mittels des prepFAST® Gerätes (Fa. Elemental Scientific).

**Tag5**

- Die nach Elementen getrennten Proben werden aus den Röhrchen wieder in die Becher überführt und zum Eintrocknen auf die Heizplatte gestellt.
- Nach Eintrocknung werden diese mit 1ml $HNO_3$ und 0.5ml $H_2O_2$ erneut aufgenommen und für weitere 3 Stunden geschlossen gekocht.

**Tag 6**

- Die Becher mit den Proben werden geöffnet und eingetrocknet.
- Die Proben werden in 10 ml $HNO_3$ aufgenommen und 4 Stunden zur Equilibrierung stehen gelassen.
- Die Proben werden in die Röhrchen für die massenspektrometrische Messung an der Neptune® (Plasmamassenspektrometer, Fa. ThermoFisher) überführt und dort gemessen.

Zur Bestimmung der Isotopenverhältnisse und/oder Verhältnisse von Erdalkalielementen können neben den massenspektrometrischen Verfahren auch spektroskopische Verfahren verwendet werden, welche mittels Lichtemission die Masseabhängigkeit der Hyperfeinstruktur der Spektrallinien nutzen. Die Allgemeingültigkeit des Verfahrens nicht einschränkend wird im Folgenden die massenspektroskopische Ermittlung der Werte beschrieben.

**Massenspektrometrische Messung:**

Diese Lösung wird in der Regel in einem Plasma-Massenspektrometer (MC-ICP-MS: Multi-Collector-Ionization Coupled - Mass-spectrometer) (z.B. ThermoFisher, Neptune) gemessen (Dieses Verfahren ist im Folgenden kurz beschrieben), können alternativ aber auch in einem Thermionenmassenspektrometer gemessen werden. Das Ziel ist es die Calciumisotopenzusammensetzung in Blutserum und/oder Urin zu bestimmen. Im TIMS werden $^{44}Ca/^{40}Ca$ Verhältnisse und in dem MC-ICP-MS $^{44}Ca/^{42}Ca$ oder alternative Verhältnisse gemessen. Beide Isotopenverhältnisse sind in ihrer Aussage gleichwertig. Die Werte unterscheiden sich nur durch einen Faktor: $^{44}Ca/^{40}Ca \cong 2.05 \cdot {}^{44}Ca/^{42}Ca$.

**Bestimmung der Calciumisotopenzusammensetzung $^{44}Ca/^{42}Ca$ unter Verwendung eines Plasma-Masspektrometers:**

Calciumisotopenmessungen werden an einem MC-ICPMS (Neptune®, Thermo Fisher Scientific) durchgeführt. Der Massenspektrometer ist mit neun Faraday-Bechern ausgestattet, von denen acht beweglich sind. Diese werden so eingerichtet, dass die atomaren Massen (u) 42, 43, 43.5 und 44 simultan gemessen werden können. Um störende Ca- und Ar-Hydride (z. B. $^{40}Ar_1H_2$ auf $^{42}Ca$) zu unterdrücken, wird ein Probeneinführungssystem APEX IR (Elemental Scientific®) verwendet. Alle Messungen werden in mittlerer Auflösung (m/$\Delta$m ~4000) auf dem störungsfreien Plateau der massearmen Seite des zentralen Mess-Peaks durchgeführt. Dies wird erreicht, indem eine geeignete mittlere Bechermasse von 4.687$\pm$0.001 u ausgewählt und täglich verifiziert wird.

Die instrumentelle Fraktionierung ("Mass-bias") wird durch Anwendung des "Standard-Sample-Bracketing, (SSB)" Verfahrens korrigiert. Die Messung einer Probe wird durch Messungen einer 5 μg/ml Ca Lösung, die aus einer 10000 μg/ml Ca-ICP-Standardlösung hergestellt wurde, korrigiert. Jede Probe wird mindestens vier Mal während einer Sitzung gemessen und deren Durchschnittswert für das weitere Vorgehen verwendet. Die Ca-Isotopenzusammensetzung wird als $\delta^{44/42}Ca$ in Teilen pro Tausend (‰) angegeben:

$$\delta^{44/42}Ca\ (‰) = [({}^{44}Ca/{}^{42}Ca)_{Probe} / ({}^{44}Ca/{}^{42}Ca)_{Referenz}] - 1$$

Die gemessene Ca-ICP-Standardlösung dient als primäres Referenzmaterial. Die $\delta^{44/42}Ca_{ICP}$-Werte werden dann unter Verwendung des gemessenen $\delta^{44/42}Ca_{ICP}$-Wertes von NIST SRM 915a in NIST SRM 915a umgewandelt: $\delta^{44/42}Ca_{SRM915a}$ (Probe, ‰ = $\delta^{44/42}Ca_{ICP}$ (Probe) - $\delta^{44/42}Ca_{ICP}$ (SRM 915a), d.h. alle Messwerte werden relative zum internationalen Standard SRM915a angegeben.

Während jeder Sitzung wird zu Beginn und am Ende jeder Sitzung chemisch unverarbeitetes NIST SRM 915a Material gemessen und diese Ergebnisse mit denen von chemisch bearbeitetem NIST SRM 915a verglichen. Der durchschnittliche Unterschied zwischen prozessiertem und unverarbeitetem SRM 915a soll in der Regel weniger als 0.01 ‰ so dass die Ca-Isotopenfraktionierung während der chemischen Reinigung vernachlässigbar angesehen werden kann.

Für die Hintergrundkorrektur wird der "On-Peak"-Ansatz verwendet. Die gemessenen Intensitäten einer 1% $HNO_3$ -Lösung werden von den Intensitäten der nachfolgenden Probenmessungen subtrahiert.

Zusätzlich werden die Messungen auf Reste von doppelt geladenem Strontium ($^{84}Sr$, $^{86}Sr$ und $^{88}Sr$) überprüft, um die korrekte Messung der Intensitäten der Massen von $^{42}Ca$, $^{43}Ca$ und $^{44}Ca$ sicherzustellen. Dazu wird zu Beginn jeder Sitzung die Intensitätsverhältnisse der Massen 42/43.5, 43/43.5 und 44/43.5 einer 2 μg/ml Sr - Lösung gemessen. Diese Verhältnisse werden dann verwendet, um die Intensitäten von doppelt geladenem Sr aus den gemessenen Intensitäten der Masse 43.5 einer gegebenen Probe zu berechnen.

Zur Qualitätssicherung werden die während der Messphase gemessenen $\delta^{44/42}Ca$-Werte von NIST SRM 1486 und IAPSO Meerwasserstandard mit publizierten Werten verglichen. Die Langzeit-Reproduzierbarkeit (2 SD = Standardabweichung) über einen Zeitraum von etwa zwei Monaten ist in der Regel besser als 0.06 ‰ für alle analysierten Referenzmaterialien.

Zur weiteren Qualitätssicherung werden mehrere Kriterien angewendet, um Daten einer einzelnen Messung, einer einzelnen Probe und ganzer Sequenzen abzulehnen. Eine einzelne Messung oder Sequenz wird verworfen, wenn:

- $|\delta^{44/42}Ca - 2\cdot\delta^{44/42}Ca| > 0.2$ ‰ gilt.

- Eine Probenmessung wird verworfen, wenn die durchschnittliche Intensität außerhalb eines Intensitätsfensters von 70 bis 130 % liegt, verglichen mit der durchschnittlichen Intensität der 5μg/ml Ca ICP -Lösung oder NIST SRM 915a-Lösung derselben Charge.

- Eine vollständige Sequenz wird abgelehnt, wenn mehr als eines der gemessenen internationalen Referenzmaterialien mehr als 0.2 ‰ vom Literaturwert entfernt ist oder die Daten nicht entlang der massenabhängigen Fraktionierungslinie fielen.

Das Ca/Sr Verhältnis wird mittels eines Standardverfahrens an einem Quadrupolmassenspektrometer bestimmt (das Verfahren ist im Folgenden beschrieben).

### Bestimmung der Ca/Sr und Mg/Ca Elementverhältnisse

Vor der Ca-Isotopenanalyse werden die Ca -, Mg - und Sr -Konzentrationen der Probenlösungen auf einem Quadrupol-Massenspekrometer (Agilent 7500cx®) unter Verwendung einer matrixangepassten externen Kalibrierungskurve und Indium als internem Standard gemessen. Während der Messung wird die Intensität der doppelt geladenen Ionen unter 2 % gehalten, um Interferenzen mit doppelt geladenem $^{88}Sr$, $^{86}Sr$ und $^{84}Sr$ auf $^{44}Ca$, $^{43}Ca$ und $^{42}Ca$ zu minimieren. Der gesamte Blank der Lösung liegt in der Regel unter 50 ng. Die Langzeit-Reproduzierbarkeit der Konzentrationsmessungen und deren Verhältnisse unter Verwendung von international verfügbaren Standards ist in der Regel besser als 5% (1 SD = Standardabweichung).

### Auswertung der Calcium-Isotopendaten:

Die gemessenen Calcium Isotopenwerte werden in der üblichen $\delta$-Notation berichtet:

$$\delta^{44/42}Ca = \left[ \frac{\left( \frac{^{44}Ca}{^{42}Ca} \right)_{Probe}}{\left( \frac{^{44}Ca}{^{42}Ca} \right)_{Standard}} - 1 \right] \cdot 1000$$

Wobei $(^{44}Ca/^{42}Ca)_{Probe}$ das im Massenspektrometer gemessene Verhältnis der Blutprobe ist und $(^{44}Ca/^{42}Ca)_{Standard}$ das Calcium Isotopenverhältnis eines international bekannten und zertifizierten Verhältnisses ist. Diese Vorgehensweise erlaubt eine internationale Vergleichbarkeit der gemessenen Isotopenverhältnisse.

### Bestimmung der Schwellenwerte:

In einer klinischen Studie (Osteogeo-Studie) wurden statistisch signifikante Schwellenwerte für die Unterscheidung von Frauen für $\delta^{44/42}Ca_{Blut}$, $\delta^{44/42}Ca_{Urin}$ und $(Ca/Sr)_{Urin}$ im Rahmen von ROC (Receiver-Operating-Characteristics) Analysen ermittelt. Um der Definition von Osteoporose entsprechend den gültigen internationalen (WHO World Health Organisation) und nationalen (DVO Dachverband für Osteoporose) Bestimmungen gerecht zu werden mussten von den Studienteilnehmerinnen diejenigen ausgeschlossen werden, welche eine Vitamin D Mangelerscheinung ($\leq 25$ $\mu$mol/l) aufwiesen. Dies betraf 20 Frauen, so dass für die Studie selbst noch 80 die Standardkriterien für Osteoporose erfüllenden Frauen übrig blieben. Von diesen 80 Frauen wurden anhand des Röntgenbefundes (DXA, golden Standard) 66 als ohne Osteoporose Befund charakterisiert und 14 als Osteoporose erkrank diagnostiziert.

Tabelle 2: ROC ermittelte Schwellenwerte:

| Mit Vitamin D Mangel* | Schwellenwert (‰) | Sensitivität (%) | Spezifität (%) | L⁻; Negativer Likelihoodfaktor | L⁺; Positiver Likelihoodfaktor |
|---|---|---|---|---|---|
| $\delta^{44/42}Ca_{Blut}$ | -0.85±0.06 | 100.0 | 55 | 0 | 2.2 (1.69 - 2.87) |
| $\delta^{44/42}Ca_{Urin}$ | 0.16±0.06 | 78.6 | 71.2 | 0.3(0.11 - 0.83) | 2.73(1.71 - 4.36) |
| $[Ca/Sr]_{Urin}$ | 2027±250 | 63.3 | 75.8 | 0.47(0.23 - 0.97) | 2.65(1.49 - 4.73) |
| **Alle** | **Schwellenwert** (‰) | **Sensitivität** (%) | **Spezifität** (%) | **L⁻; Negativer Likelihoodfaktor** | **L⁺; Positiver Likelihoodfaktor** |
| $\delta^{44/42}Ca_{Blut}$ | -0.85±0.06 | 94.4 | 53.7 | 0.10 (0.02 - 0.70) | 2.04 (1.57 - 2.64) |
| $\delta^{44/42}Ca_{Urin}$ | 0.23±0.06 | 72.2 | 67.1 | 0.41 (0.19 - 0.89) | 2.19 (1.44 - 3.34) |
| $[Ca/Sr]_{Urin}$ | 1772±250 | 72.2 | 61.0 | 0.46 (0.21- 0.98) | 1.85 (1.25 - 2.75) |
| **Bemerkung:** *Vitamin D ($\leq 25$ $\mu$mol/l). | | | | | |

Der negative Likelihoodfaktor (L⁻) gibt die Wahrscheinlichkeit für einen Wert über dem Schwellenwert an einen positiven Osteoporose Befund relativ zu einer gesunden Person zu bekommen.

Der positive Likelihoodfaktor (L⁺) gibt die Wahrscheinlichkeit für einen Wert unterhalb des Schwellenwertes an einen positiven Osteoporose Befund relativ zu einer gesunden Person zu bekommen.

**Exemplarisches Vorgehen bei der Erfindungsgemäßen Diagnose anhand einer Blut ($\delta^{44/42}Ca_{Blut}$)- und Urinprobe ($\delta^{44/42}Ca_{Blut}$)**

Eingedenk der großen Ähnlichkeit der Werte (Tabelle 2) wurden alle 100 Probandinnen der Osteogeo-Studie ohne Einschränkung durch die Vitamin D Konzentration betrachtet. Gleichwohl erhöht sich die Sensitivität der Diagnose wenn die Vitamin D Konzentration bekannt ist (Tabelle 2).

Die statistische ROC Analyse ergab für die gesamte Studiengruppe einen Schwellenwert für $\delta^{44/42}Ca_{Blut}$ von **-0.85±0.06 ‰** ($\delta^{44/42}Ca_{Schwellenwert-Blut}$). Die Sensitivität des Verfahrens wurde mit 94.4 % errechnet und die Spezifität beträgt 53.7 % (Tabelle 2). Im Blut gemessene Werte gesunder Probanden die oberhalb dieses Schwellenwertes liegen haben nur eine 0.1 fache Wahrscheinlichkeit einen positiven Befund (Osteoporose) zu bekommen (negativer Likelihoodfaktor L⁻ = 0.1). Gemessene Calcium Isotopenwerte unterhalb des Schwellenwertes haben eine 2.04 fach höhere Wahrscheinlichkeit das eine kranke Person (positiver Likelihoodfaktor L⁺ = 2.04) einen positiven Befund (Osteoporose) bekommt.

**Exemplarisches Vorgehen bei der erfindungsgemäßen Osteoporose/Nierenfunktions-Diagnose für den Fall das sowohl der Calcium Isotopenwert einer Blutprobe ($\delta^{44/42}Ca_{Blut}$) als auch einer Urinprobe ($\delta^{44/42}Ca_{Urin}$) vorliegen - Abbildung 8:**

a) Bilden der Differenz $\Delta_{Urin-Blut}$ und Berechnung der renalen Reabsorbtion ($f_{renale-Reabsorbtion}$). Klassifikation der Nierenfunktion entsprechend Tabelle 1.

b) Gilt $\delta^{44/42}Ca_{Blut-gemessen} \geq \delta^{44/42}Ca_{Schwellenwert-Blut}$ + 0.06.
Im Blut gemessene Werte gesunder Probanden die oberhalb dieses Schwellenwertes einschließlich des Toleranzwertes liegen haben nur eine 0.1 fache Wahrscheinlichkeit einen positiven Osteoporose Befund zu bekommen. Die Ampel ist grün! Es liegt mit hoher Wahrscheinlichkeit **keine** Osteoporose vor (Ampel ist grün!)

c) Gilt $\delta^{44/42}Ca_{Schwellenwert-Blut}$ + 0.06 $\geq \delta^{44/42}Ca_{Blut-gemessen} \geq \delta^{44/42}Ca_{Schwellenwert-Blut}$ - 0.06 ‰.
Aufgrund der Lage innerhalb des Toleranzbereichs des Schwellenwertes wird dem Patient eine beginnende Osteoporose diagnostiziert. Die Ampel ist gelb! Diesen Patientinnen wird eine zusätzliche DXA-Messung empfohlen um für spezifische Knochen (z.B. Oberschenkel) das individuelle Risiko für einen Knochenbruch zu bestimmen.

d) Gilt $\delta^{44/42}Ca_{Blut-gemessen} \leq \delta^{44/42}Ca_{Schwellenwert-Blut}$ -0.06 ‰.
Im Blut gemessene Calcium Isotopenwerte unterhalb des Schwellenwertes einschließlich des Toleranzwertes haben eine 2.04 fach höhere Wahrscheinlichkeit einen positiven Befund (Osteoporose) bekommt.

Es liegt vermutlich ein Nettoverlust von Calcium und Osteoporose vor! Diesen Patientinnen wird eine zusätzliche DXA-Messung empfohlen um für spezifische Knochen das individuelle Risiko für einen Knochenbruch zu bestimmen.

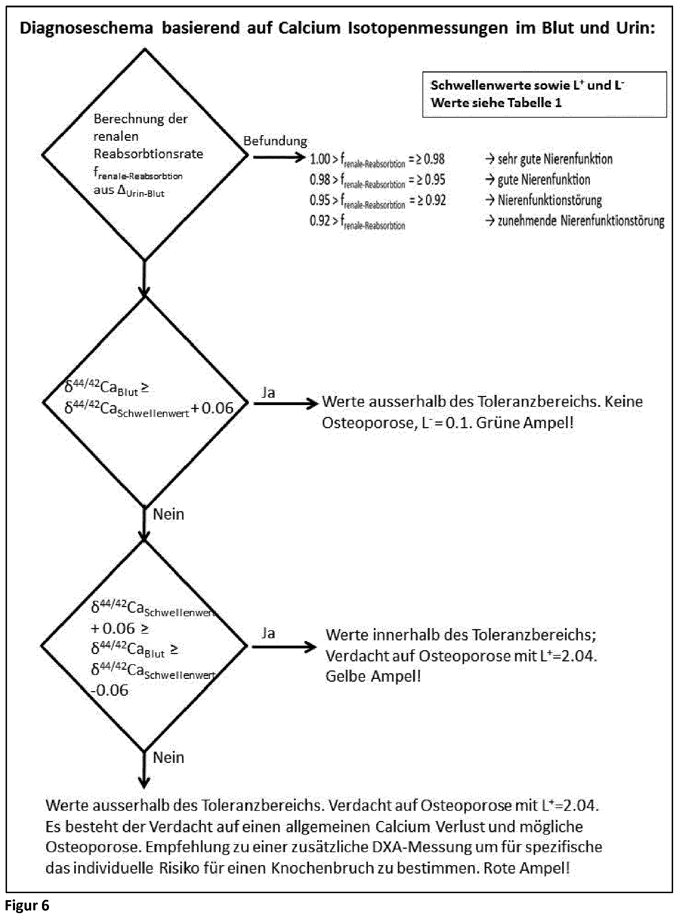

Im Folgenden findet sich eine graphische Darstellung des Diagnosepfades: (siehe Figur 6)

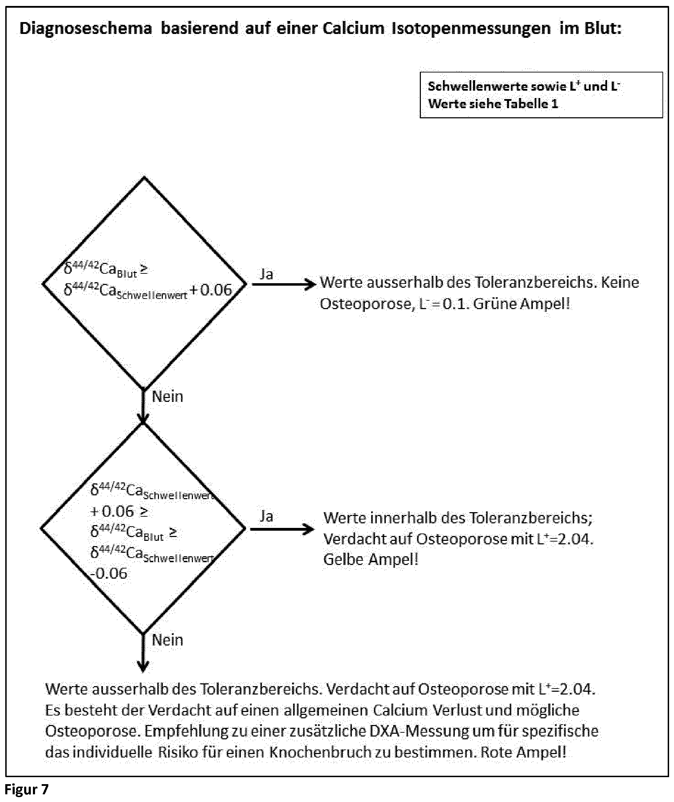

**Exemplarisches Vorgehen bei der erfindungsgemäßen Diagnose für den Fall das nur eine Blutprobe ($\delta^{44/42}Ca_{Blut}$) vorliegt (Figur 7):**

Eine Aussage über Osteoporose kann auch gemacht werden, wenn nur eine Blutprobe vorliegt. Aufgrund der höheren Sensitivität der Blutproben gegenüber der Urinprobe (Tabelle 2) ist die diagnostische Aussage einer Blutprobe höher wie die einer Urinprobe, da die Urinprobe durch eine eingeschränkte Nierenfunktion beeinträchtigt sein können. Die Sensitivität der diagnostischen Aussage erhöht sich zusätzlich, wenn die Vitamin D Konzentration der Patientin bekannt ist (Tabelle 2).

a) **Gilt $\delta^{44/42}Ca_{Blut-gemessen} \geq \delta^{44/42}Ca_{Schwellenwert-Blut}$ + 0.06:**
Gemessene Calcium Isotopenwerte oberhalb dieses Schwellenwertes einschließlich der Toleranz haben nur eine 0.1 fache Wahrscheinlichkeit einen positiven Befund (Osteoporose) zu bekommen (Die Ampel ist grün!).

b) **Gilt $\delta^{44/42}Ca_{Schwellenwert-Blut}$ + 0.06: $\geq \delta^{44/42}Ca_{Blut-gemessen} \geq \delta^{44/42}Ca_{Schwellenwert-Blut}$ - 0.06:**
Aufgrund der Lage innerhalb des Toleranzbereichs des Schwellenwertes wird dem Patient eine beginnende Osteoporose diagnostiziert (Die Ampel ist gelb!)! Diesen Patientinnen wird eine zusätzliche DXA-Messung empfohlen um für spezifische Knochen das individuelle Risiko für einen Knochenbruch zu bestimmen.

**c) Gilt $\delta^{44/42}Ca_{Blut-gemessen} \leq \delta^{44/42}Ca_{Schwellenwert-Blut}$- 0.06:**
Gemessene Ca Isotopenwerte unterhalb des Schwellenwertes einschließlich der Toleranz haben eine 2.04 fach höhere Wahrscheinlichkeit einen positiven Befund (Osteoporose) zu bekommen (Die Ampel ist rot!). Es besteht der Verdacht auf einen allgemeinen Calcium Verlust und mögliche Osteoporose. Diesen Patientinnen wird eine zusätzliche DXA-Messung empfohlen um für spezifische Knochen das individuelle Risiko für einen Knochenbruch zu bestimmen.

**Exemplarisches Vorgehen bei der erfindungsgemäßen Diagnose für den Fall das nur eine Urinprobe ($\delta^{44/42}Ca_{Urin}$) vorliegt (Figur 8):**

Eine Aussage über Osteoporose kann auch gemacht werden, wenn nur eine Urinprobe vorliegt. Die Sensitivität der diagnostischen Aussage erhöht sich, wenn die Vitamin D Konzentration der Patientin bekannt ist.

**a) Gilt $\delta^{44/42}Ca_{Urin-gemessen} \geq \delta^{44/42}Ca_{Schwellenwert-Urin}$ + 0.06:**
Gemessene Calcium Isotopenwerte oberhalb dieses Schwellenwertes einschließlich der Toleranz haben nur eine 0.41 fache Wahrscheinlichkeit einen positiven Befund (Osteoporose) zu bekommen (Die Ampel ist grün!).

**b) Gilt $\delta^{44/42}Ca_{Schwellenwert-Urin}$, + 0.06: $\geq \delta^{44/42}Ca_{Urin-gemessen} \geq \delta^{44/42}Ca_{Schwellenwert-Urin}$ - 0.06:**
Aufgrund der Lage innerhalb des Toleranzbereichs des Schwellenwertes wird dem Patient eine beginnende Osteoporose diagnostiziert (Die Ampel ist gelb!)! Diesen Patientinnen wird eine zusätzliche DXA-Messung empfohlen um für spezifische Knochen das individuelle Risiko für einen Knochenbruch zu bestimmen.

**c) Gilt $\delta^{44/42}Ca_{Urin-gemessen} \leq \delta^{44/42}Ca_{Schwellenwert-Urin}$- 0.06:**
Gemessene Ca Isotopenwerte unterhalb des Schwellenwertes einschließlich der Toleranz haben eine 2.19 fach höhere Wahrscheinlichkeit einen positiven Befund (Osteoporose) zu bekommen (Die Ampel ist rot!). Es besteht der Verdacht auf einen allgemeinen Calcium Verlust und mögliche Osteoporose. Diesen Patientinnen wird eine zusätzliche DXA-Messung empfohlen um für spezifische Knochen das individuelle Risiko für einen Knochenbruch zu bestimmen.

Figur 9:     Calcium Isotopie der Nahrung, des Stuhls, des Blutes und des Urin bei den postmenopausalen Frauen.

Figur 10:    Zusammenhang zwischen dem isotopischen Unterschied von Blut und Urin ($\Delta^{44/42}Ca_{Blut-Urin}$) sowie der Rate der renalen Reabsorbtion ($f_{renale-Reabsorbtion}$) als direktes Maß für die Nierenfunktion. Zusätzliche sind die definierten Bereiche unterschiedlicher Nierenfunktionen (Schwellenwerte) dargestellt.

Die Erfindung betrifft weiterhin ein Testkit zur Verwendung in einem Verfahren zur Diagnose einer Erkrankung, die mit einer verminderten Knochendichte und/oder Calciumverlust einhergeht, umfassend:

-    eine Verpackung, diese umfassend:
-    einen Auffangbehälter zur Aufnahme der Probenflüssigkeit,
-    mindestens eine Monovette zur Aufnahme eines Teils der Probenflüssigkeit und zum Transport zu einem Messgerät, das Isotopenverhältnisse und/oder Mengenverhältnisse von Erdalkalimetall-Anteilen bestimmt, und
-    eine Anleitung,

wobei die Anleitung einen festen vom Individuum unabhängigen Schwellenwert für das Isotopenverhältnis und/oder das Mengenverhältnis der Erdalkalimetall-Anteile zum Vergleich mit dem oder den zu ermittelnden Probenwerten ausweist.
Ein fester Schwellenwert für das Isotopenverhältnis $^{44}Ca/^{42}Ca$ nach einer $\delta$-Notation insbesondere von $\delta^{44/42}Ca_{Urin}$ = 0,23$\pm$0.06 ‰ für einen Urintestkit oder $\delta^{44/42}Ca_{Blut}$ = - 0.85$\pm$0.06 ‰ für einen Bluttestkit kann in der Anleitung des Testkits ausgewiesen sein.
Weiterhin betrifft die vorliegende Erfindung ein Testkit zur Verwendung in einem erfindungsgemäßen Verfahren, umfassend

-    eine Verpackung, diese umfassend:
-    einen Auffangbehälter zur Aufnahme der Probenflüssigkeit,
-    mindestens eine Monovette zur Aufnahme eines Teils der Probenflüssigkeit und zum Transport zu einem Messgerät, das Isotopenverhältnisse und/oder Mengenverhältnisse von Erdalkalimetall-Anteilen bestimmt, und
-    eine Anleitung, wobei ein von einem oder mehreren durch den individuellen Patienten bedingten Faktoren unabhängiger Schwellenwert zum Vergleich des mindestens einen Probenwertes mit dem Schwellenwert in dem Testkit angegeben ist.

Ein solcher Testkit kann alle für das Verfahren vorstehend genannten Merkmale umfassen beziehungsweise alle für das erfindungsgemäße Verfahren und seine Ausgestaltungen, einzeln oder in Kombination, notwenigen Einrichtungen aufweisen.

Vorteilhaft an dem erfindungsgemäßen Testkit ist insbesondere, dass erstmals eine einfache Probe ausreicht, um eine Basis für eine Diagnose in Bezug auf eine Erkrankung, die mit einer verminderten Knochendichte und/oder Calcium Verlust einhergeht, zu stellen. Ein Vergleich mit dem festen, insbesondere vom Patienten unabhängige weitere Werte, Schwellenwert stellt eine sehr große Vereinfachung der Abläufe der bisher bekannten Diagnosekits dar.

## Patentansprüche

1. Verfahren zur Diagnose einer Erkrankung, die mit einer verminderten Knochendichte und/oder Calcium Verlust einhergeht, umfassend folgende Schritte:

   a) Bestimmen von Isotopenverhältnissen oder Mengenverhältnissen von Erdalkalimetall-Anteilen in einer Probe von Urin oder Blut oder Stuhl,
   b) hierdurch Ermitteln mindestens eines Probenwertes, und
   c) Vergleich des mindestens einen Probenwertes mit einem Schwellenwert, **dadurch gekennzeichnet, dass** der Schwellenwert unabhängig gewählt ist von einem oder mehreren durch den individuellen Patienten bedingten Faktoren.

2. Verfahren nach Anspruch 1, **dadurch gekennzeichnet, dass** in Schritt a) die Isotopenverhältnisse oder Mengenverhältnisse der Erdalkalimetall-Anteile in der Probe massenspektrometrisch bestimmt werden.

3. Verfahren nach Anspruch 1 oder 2, **dadurch gekennzeichnet, dass** in Schritt a) die Isotopenverhältnisse des Calciums (Ca) bestimmt werden.

4. Verfahren nach einem der vorhergehenden Ansprüche, **dadurch gekennzeichnet, dass** in Schritt a) das Mengenverhältnis der Erdalkalielemente von Calcium (Ca) zu Strontium (Sr) bestimmt wird.

5. Verfahren nach einem der vorhergehenden Ansprüche, **dadurch gekennzeichnet, dass** in Schritt c) ein vom Individuum unabhängiger fester Schwellenwert, welcher je nach Probenart vorher festgelegt wurde, als Vergleichswert dient.

6. Verfahren nach einem der vorhergehenden Ansprüche, **dadurch gekennzeichnet, dass** der Schwellenwert in Schritt c) für das Isotopenverhältnis $^{44}Ca/^{42}Ca$ in einer Blutprobe nach einer $\delta$-Notation bei $\delta^{44/42}Ca_{Blut} = -0{,}85 \pm 0{,}06$ ‰ liegt.

7. Verfahren nach einem der vorhergehenden Ansprüche, **dadurch gekennzeichnet, dass** der Schwellenwert in Schritt c) für das Isotopenverhältnis $^{44}Ca/^{42}Ca$ in einer Urinprobe nach einer $\delta$-Notation bei $\delta^{44/42}Ca_{Urin} = 0{,}23 \pm 0{,}06$ ‰ liegt.

8. Verfahren nach Anspruch 6, **dadurch gekennzeichnet, dass** der Schwellenwert entsprechend der Formel:

$$\delta^{m3/m2}Ca = \delta^{m3/m1}Ca \times \left[\frac{ln(m3/m2)}{ln(m3/m1)}\right]$$

   mit m3 > m2 > m1, wobei m3, m2, m1 die Massenzahlen repräsentieren, auf andere Isotopenverhältnisse des Calciums übertragbar ist.

9. Verfahren nach einem der vorhergehenden Ansprüche, **dadurch gekennzeichnet, dass** der Schwellenwert in Schritt c) für das Mengenverhältnis der Erdalkalielemente von Calcium (Ca) zu Strontium (Sr) bei $1772 \pm 250$ $mol_{Ca}/mol_{Sr}$ liegt.

10. Verfahren nach einem der vorhergehenden Ansprüche, **dadurch gekennzeichnet, dass** in Schritt b) ein Probenwert als $\delta^{44/42}Ca_{Urin}$ und ein weiterer Probenwert als $\delta^{44/42}Ca_{Blut}$ ermittelt werden und in Schritt c) die Differenz der beiden Probenwerte mit einem Schwellenwert vergleichen wird, um zusätzlich eine Aussage über die Nierenfunktion zu treffen.

11. Verfahren nach einem der vorhergehenden Ansprüche, **dadurch gekennzeichnet, dass** in Schritt b) ein Probenwert als $\delta^{44/42}Ca_{Stuhl}$ und ein weiterer Probenwert als $\delta^{44/42}Ca_{Blut}$ ermittelt werden und in Schritt c) die Differenz der beiden Probenwerte mit einem Schwellenwert vergleichen wird, um zusätzlich eine Aussage über die Darmfunktion zu treffen.

12. Verfahren nach einem der vorhergehenden Ansprüche, **dadurch gekennzeichnet, dass** es sich bei Erkrankung, die mit einer verminderten Knochendichte und/oder Calcium Verlust einhergeht, um Osteoporose, Osteomalazie, Multiples Myelom und/oder Nierenfunktionsstörung handelt.

13. Verfahren nach einem der vorhergehenden Ansprüche, **dadurch gekennzeichnet, dass** die Mengenverhältnisse in Schritt a) als Molverhältnisse der Erdalkalimetall-Anteile in der Probe oder als Massenverhältnisse der Erdalkalimetall-Anteile in der Probe bestimmt werden.

14. Verfahren nach einem der vorhergehenden Ansprüche, **dadurch gekennzeichnet, dass** wenn in Schritt b) ein Probenwert als $\delta^{44/42}Ca_{Urin}$ bestimmt wird und in Schritt c) der Vergleich mit dem Schwellenwert nach einer $\delta$-Notation von $\delta^{44/42}Ca_{Urin} = 0{,}23 \pm 0.06\ ‰$ ergibt, dass der Probenwert größer ist, so kann das Vorliegen einer Osteoporose-Erkrankung als unwahrscheinlich ausgeschlossen werden.

15. Verfahren nach einem der vorhergehenden Ansprüche 1-13, **dadurch gekennzeichnet, dass** wenn in Schritt b) ein Probenwert als $\delta^{44/42}Ca_{Blut}$ bestimmt wird und in Schritt c) der Vergleich mit dem Schwellenwert nach einer $\delta$-Notation von $\delta^{44/42}Ca_{Blut} = -0.85 \pm 0.06\ ‰$ ergibt, dass der Probenwert größer ist, so kann das Vorliegen einer Osteoporose-Erkrankung als unwahrscheinlich ausgeschlossen werden.

16. Testkit zur Verwendung in einem Verfahren zur Diagnose einer Erkrankung, die mit einer verminderten Knochendichte und/oder Calciumverlust einhergeht, umfassend:

    - eine Verpackung, diese umfassend:
    - einen Auffangbehälter zur Aufnahme der Probenflüssigkeit,
    - mindestens eine Monovette zur Aufnahme eines Teils der Probenflüssigkeit und zum Transport zu einem Messgerät, das Isotopenverhältnisse und/oder Mengenverhältnisse von Erdalkalimetall-Anteilen bestimmt, und
    - eine Anleitung,

    **dadurch gekennzeichnet, dass** die Anleitung einen festen vom Individuum unabhängigen Schwellenwert für das Isotopenverhältnis und/oder das Mengenverhältnis der Erdalkalimetall-Anteile zum Vergleich mit dem oder den zu ermittelnden Probenwerten ausweist.

17. Testkit nach Anspruch 16, **dadurch gekennzeichnet, dass** ein Schwellenwert für das Isotopenverhältnis 44Ca/42Ca nach einer $\delta$-Notation von $\delta44/42Ca_{Urin} = 0{,}23 \pm 0.06\ ‰$ oder $\delta44/42Ca_{Blut} = -0.85 \pm 0.06\ ‰$ ausgewiesen wird.

18. Testkit zur Verwendung in einem Verfahren gemäß einem der Ansprüche 1 bis 15, umfassend

    - eine Verpackung, diese umfassend:
    - einen Auffangbehälter zur Aufnahme der Probenflüssigkeit,
    - mindestens eine Monovette zur Aufnahme eines Teils der Probenflüssigkeit und zum Transport zu einem Messgerät, das Isotopenverhältnisse und/oder Mengenverhältnisse von Erdalkalimetall-Anteilen bestimmt, und
    - eine Anleitung, wobei ein von einem oder mehreren durch den individuellen Patienten bedingten Faktoren unabhängiger Schwellenwert zum Vergleich des mindestens einen Probenwertes mit dem Schwellenwert in dem Testkit angegeben ist.

**Figur 1**

**Figur 2**

**Diagnoseschema basierend auf Ca/Sr Elementverhältnissen im Urin:**

Figur 3

Beispielhafte Werte aus der klinischen Studie:

$$F_{renale-Reabsorbtion}$$
$$f_{renale-Reabsorbtion} = (1-f_{Urin}) \longrightarrow \text{Renale Reabsorbtion!}$$
$$\delta^{44/42}Ca_{renale-Reabsorbtion}$$

$$F_{renale-Reabsorbtion} = 6.67 \ g_{Ca}/Tag$$

$$f_{renal-Reabsorbtion} = 0.092$$

**Blut**

$\delta^{44/42}Ca = -0.84 \ ‰$

$$F_{filtration} = 6.79 \ g_{Ca}/Tag$$

**Niere**

$$F_{filtration} = F_{renale-Reabsorption} + F_{Urine}$$
$$F_{Urine} = f_{Urine} \cdot F_{filtration}$$
$$F_{renale-Reabsorption} = f_{renale-Reabsorbtion} \cdot F_{filtration}$$

$Mit: 0 < f < 1$

$$F_{Urin} = 0.12 \ g_{Ca}/Tag$$
$$\delta^{44/42}Ca_{Urin} = +0.35 \ ‰$$
$$f_{Urine} = 0.018$$

**Figur 4**

$$f_{Urin}$$

0.1          0.05          $f_{Urin} = 0.018$   0

0.50

$$\delta^{44/42}Ca_{Urin} = 0.35 \ ‰$$

0.30

0.10

**Urin**

-0.10

$\alpha$

-0.30

$$\delta^{44/42}Ca$$

-0.50

**renal-Reabsorbiert**

-0.70

-0.90

-1.10

-1.30

**Fraktionierungsfaktor: $\alpha = 0.9997$**

$$\delta^{44/42}Ca_{renale-Reabsorbtion} = -0.86 \ ‰$$

**Figur 5**

## Diagnoseschema basierend auf Calcium Isotopenmessungen im Blut und Urin:

Schwellenwerte sowie L$^+$ und L$^-$ Werte siehe Tabelle 1

Berechnung der renalen Reabsorbtionsrate $f_{renale-Reabsorbtion}$ aus $\Delta_{Urin-Blut}$

Befundung

$1.00 > f_{renale-Reabsorbtion} = \geq 0.98$ → sehr gute Nierenfunktion

$0.98 > f_{renale-Reabsorbtion} = \geq 0.95$ → gute Nierenfunktion

$0.95 > f_{renale-Reabsorbtion} = \geq 0.92$ → Nierenfunktionstörung

$0.92 > f_{renale-Reabsorbtion}$ → zunehmende Nierenfunktionstörung

$\delta^{44/42}Ca_{Blut} \geq \delta^{44/42}Ca_{Schwellenwert} + 0.06$

Ja → Werte ausserhalb des Toleranzbereichs. Keine Osteoporose, L$^-$=0.1. Grüne Ampel!

Nein

$\delta^{44/42}Ca_{Schwellenwert} + 0.06 \geq \delta^{44/42}Ca_{Blut} \geq \delta^{44/42}Ca_{Schwellenwert} -0.06$

Ja → Werte innerhalb des Toleranzbereichs; Verdacht auf Osteoporose mit L$^+$=2.04. Gelbe Ampel!

Nein

Werte ausserhalb des Toleranzbereichs. Verdacht auf Osteoporose mit L$^+$=2.04. Es besteht der Verdacht auf einen allgemeinen Calcium Verlust und mögliche Osteoporose. Empfehlung zu einer zusätzliche DXA-Messung um für spezifische das individuelle Risiko für einen Knochenbruch zu bestimmen. Rote Ampel!

Figur 6

**Diagnoseschema basierend auf einer Calcium Isotopenmessungen im Blut:**

Schwellenwerte sowie L$^+$ und L$^-$
Werte siehe Tabelle 1

$\delta^{44/42}Ca_{Blut} \geq$
$\delta^{44/42}Ca_{Schwellenwert} + 0.06$

Ja → Werte ausserhalb des Toleranzbereichs. Keine Osteoporose, L$^-$=0.1. Grüne Ampel!

Nein

$\delta^{44/42}Ca_{Schwellenwert} + 0.06 \geq$
$\delta^{44/42}Ca_{Blut} \geq$
$\delta^{44/42}Ca_{Schwellenwert} -0.06$

Ja → Werte innerhalb des Toleranzbereichs; Verdacht auf Osteoporose mit L$^+$=2.04. Gelbe Ampel!

Nein

Werte ausserhalb des Toleranzbereichs. Verdacht auf Osteoporose mit L$^+$=2.04. Es besteht der Verdacht auf einen allgemeinen Calcium Verlust und mögliche Osteoporose. Empfehlung zu einer zusätzliche DXA-Messung um für spezifische das individuelle Risiko für einen Knochenbruch zu bestimmen. Rote Ampel!

Figur 7

**Diagnoseschema basierend auf einer Calcium Isotopenmessungen im Urin:**

Schwellenwerte sowie L$^+$ und L$^-$
Werte siehe Tabelle 1

$\delta^{44/42}Ca_{Urin} \geq \delta^{44/42}Ca_{Schwellenwert} + 0.06$

**Ja** → Werte ausserhalb des Toleranzbereichs. Keine Osteoporose, L$^-$=0.1. Grüne Ampel!

**Nein**

$\delta^{44/42}Ca_{Schwellenwert} + 0.06 \geq \delta^{44/42}Ca_{Urin} \geq \delta^{44/42}Ca_{Schwellenwert} - 0.06$

**Ja** → Werte innerhalb des Toleranzbereichs; Verdacht auf Osteoporose mit L$^+$=2.04. Gelbe Ampel!

**Nein**

Werte ausserhalb des Toleranzbereichs. Verdacht auf Osteoporose mit L$^+$=2.04. Es besteht der Verdacht auf einen allgemeinen Calcium Verlust und mögliche Osteoporose. Empfehlung zu einer zusätzliche DXA-Messung um für spezifische das individuelle Risiko für einen Knochenbruch zu bestimmen. Rote Ampel!

**Figur 8**

Figur 9

Figur 10

**Europäisches Patentamt**
**European Patent Office**
**Office européen des brevets**

# EUROPÄISCHER RECHERCHENBERICHT

**Nummer der Anmeldung**

EP 18 19 1474

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | G W GORDON ET AL: "Predicting multiple myeloma disease activity by analyzing natural calcium isotopic composition", LEUKEMIA., Bd. 28, Nr. 10, 12. Juni 2014 (2014-06-12), Seiten 2112-2115, XP055529966, US ISSN: 0887-6924, DOI: 10.1038/leu.2014.193 * das ganze Dokument * * insbesondere Fig. 1 a,b,c sowie S. 2114, Sp. 1, 2. Par. * ----- | 1-3,5, 12,16-18 | INV. G01N33/84 |
| X | US 2012/225486 A1 (HILLEGONDS DARREN J [US] ET AL) 6. September 2012 (2012-09-06) * das ganze Dokument * ----- | 1-3,5, 12,13, 16-18 | |
| X,D | US 2013/115650 A1 (ANBAR ARIEL [US] ET AL) 9. Mai 2013 (2013-05-09) * das ganze Dokument * * insbesondere Anspruch 14 * ----- | 1-5,12, 13,16-18 | |
| X,D | US 2014/273248 A1 (ANBAR ARIEL [US] ET AL) 18. September 2014 (2014-09-18) * das ganze Dokument * * Abbildungen 1A-D,4 * ----- | 1-3,5, 16-18 | **RECHERCHIERTE SACHGEBIETE (IPC)** G01N |

Der vorliegende Recherchenbericht wurde für alle Patentansprüche erstellt

| Recherchenort | Abschlußdatum der Recherche | Prüfer |
|---|---|---|
| München | 5. Dezember 2018 | Hoesel, Heidi |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 18 19 1474

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-12-2018

| Im Recherchenbericht angeführtes Patentdokument | Datum der Veröffentlichung | Mitglied(er) der Patentfamilie | Datum der Veröffentlichung |
|---|---|---|---|
| US 2012225486 A1 | 06-09-2012 | US 2006115427 A1<br>US 2012225486 A1 | 01-06-2006<br>06-09-2012 |
| US 2013115650 A1 | 09-05-2013 | US 2013115650 A1<br>WO 2011156583 A1 | 09-05-2013<br>15-12-2011 |
| US 2014273248 A1 | 18-09-2014 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20130115650 A1

- US 20140273248 A1

**In der Beschreibung aufgeführte Nicht-Patentliteratur**

- **SKULAN, JOSEPH et al.** Natural Calcium Isotopic Composition of Urine as a Marker of Bone Mineral Balance. *Clinical Chemistry,* 2007, vol. 53, 1155-1158
- **SAULO KLAHR ; ANDREW S. LEVEY ; GERALD J. BECK ; ARLENE W. CAGGIULA ; LAWRENCE HUNSICKER ; JOHN W. KUSEK ; GARY STRIKER.** The Modification of Diet in Renal Disease Study Group: The Effects of Dietary Protein Restriction and Blood-Pressure Control on the Progression of Chronic Renal Disease. *N Engl J Med.,* 31. Marz 1994, vol. 330 (13), 877-884

- **STEVENS, LESLEY A. et al.** Evaluation of the Modification of Diet in Renal Disease Study Equation in a Large Diverse Population. *J Am Soc Nephrol,* 2007, 2749-2757
- Nephrology beyond JASN. Eberhard Ritz Feature Editor: Estimated GFR: Are There Limits to Its Utility?. J Am Soc Nephrol. 2006, vol. 17, 2077-2085
- The Calcium Economy. **HEANEY RP.** Calcium in Human Health. Human Press, 2006, 145-162